# EUROPEAN PATENT SPECIFICATION

(11) **EP 2 266 473 B1**
(45) Date of publication and mention of the grant of the patent: **15.07.2015**
(21) Application number: 09730620.3
(22) Date of filing: 06.04.2009
(51) Int. Cl.: A61B 17/12, A61M 25/01

(54) **LINEAR OBJECT OPERATION CONTROLLER WHICH CONTROLS OPERATION OF LINEAR OBJECT BY OPERATOR**
BEDIENUNGSSTEUERUNG FÜR LINEARES OBJEKT ZUR STEUERUNG DES BETRIEBS EINES LINEAREN OBJEKTS DURCH EINEN BEDIENER
CONTRÔLEUR D'ACTIONNEMENT D'OBJET LINÉAIRE QUI COMMANDE L'ACTIONNEMENT D'UN OBJET LINÉAIRE PAR UN OPÉRATEUR

(30) Priority: 10.04.2008 JP 2008102440; 19.05.2008 JP 2008130536; 29.05.2008 JP 2008140991
(43) Date of publication of application: 29.12.2010
(73) Proprietor: NTN Corporation, Osaka-shi, Osaka 550-0003 (JP)
(72) Inventor: NAGANO, Yoshitaka, Iwata-shi Shizuoka 438-8510 (JP); NISHIO, Yukihiro, Iwata-shi Shizuoka 438-8510 (JP); OZAKI, Takayoshi, Iwata-shi Shizuoka 438-8510 (JP)
(74) Representative: Bockhorni & Kollegen
(86) International application number: PCT/JP2009/057062
(87) International publication number: WO 2009/125744

(56) References cited:
- WO-A1-2009/144995
- JP-A- 2000 042 116
- JP-A- 2001 157 662
- JP-T- 8 506 512
- US-A1- 2005 234 293
- US-A1- 2008 009 791

## Description

### TECHNICAL FIELD

The present invention relates to linear object manipulation control devices, particularly a linear object manipulation control device for controlling manipulation of a linear object such as a catheter or delivery wire inserted into a body.

### BACKGROUND ART

As surgical procedures to prevent the rupture of a cerebral aneurysm that is the main cause of subarachnoid bleeding, treatment using a minimally invasive catheter is now carried out. This treatment is called coil embolization. Specifically, a catheter is inserted from the artery of the thigh up to the cerebral aneurysm. A delivery wire with a platinum coil at the tip is inserted through the catheter to allow the cerebral aneurysm to be filled with the platinum coil.

Among the several methods of detaching the platinum coil from the delivery wire, the electrical detaching method is widely employed. In the case of the electrical type, the platinum coil is connected to the delivery wire with an electrolyzing material. By applying voltage across the syringe needle pierced into the body and the delivery wire, the connecting region is electrolyzed such that the coil is left in the cerebral aneurysm. One such example is disclosed in Patent Document 1 (Japanese National Patent Publication No. 8-506512). According to Patent Document 1 (Japanese National Patent Publication No. 8-506512), there are provided an electrode electrically connected to the skin, and an electrolyzing link (connecting portion).

The coil embolization is carried out generally according to the following procedures. (1) Two catheters (primary catheter and secondary catheter) and a guide wire are inserted into the artery of the thigh. The primary catheter has the secondary catheter placed therein. The guide wire is inserted in the secondary catheter. The leading end of the secondary catheter is guided by the guide wire to be located in the cerebral aneurysm. (2) The guide wire is withdrawn from the secondary catheter. A delivery wire with a platinum coil (hereinafter, referred to as "coil") at the tip is then inserted into the secondary catheter in lieu of the guide wire. (3) After the coil is placed in the cerebral aneurysm, an electrode is connected to the delivery wire. An electrode is also connected to the needle that has already been pierced into the body. Subsequently, a current is conducted across the delivery wire and the body via these electrodes. Since the coil and the delivery wire are connected through an electrolyzing material, the coil is detached from the delivery wire by the electrical conduction. As a result, the coil is left in the cerebral aneurysm. Then, (4) the delivery wire is drawn out from the secondary catheter, and a delivery wire with another coil attached is inserted into the secondary catheter. (5) The steps of (3) to (4) are repeated until the cerebral aneurysm is densely filled with coil.

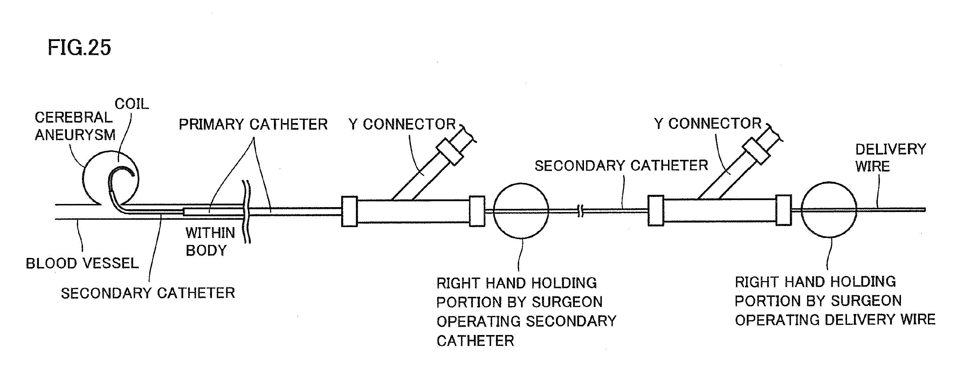

In the coil embolization, the cerebral aneurysm must be filled as densely as possible. During the manipulation by a surgeon to insert a coil into the body, the surgeon controls the tip of the secondary catheter and the position of the coil by manipulating the secondary catheter and delivery wire at the entrance of a Y connector, as shown in Fig. 25. The Y connector is employed to introduce externally applied physiological saline for the purpose of reducing the friction between the catheter and delivery wire, or to introduce an externally applied angiography agent to a target site in the body after the catheter inserted into the vessel is guided from outside the body to the target site, for example.

The force of inserting the delivery wire required to place a coil (the compressive force along the longitudinal axis acting on the linear object) will become greater as the filling with a coil advances. However, the insertion force cannot be raised higher than a predetermined level since the risk of rupture of the cerebral aneurysm will become higher if the insertion force is increased.

In this case, the secondary catheter is manipulated to move back and forth to cause the tip of the secondary catheter to move to a space in the cerebral aneurysm stuffed with coil. Accordingly, a coil can be introduced into a space. Thus, the insertion force of the delivery wire required for placing a coil into a space can be reduced. If the insertion force increases, the manipulation of shifting the tip of the secondary catheter is carried out again to allow the cerebral aneurysm to be filled with coil sufficiently through an insertion force as small as possible.

In manipulating the secondary catheter to move back and forth, conjunction with the manipulation of moving the delivery wire back and forth is required. The surgeon will manipulate the second catheter and delivery wire while confirming the movement of the tip of the secondary catheter and coil through an X-ray image. Generally, the moving direction by the manipulation of the secondary catheter and delivery wire is in opposite directions. When the tip of the secondary catheter is to be moved so as to be extracted from the cerebral aneurysm, the surgeon exerts a force on the delivery wire in a direction so as to insert the coil, already placed in the cerebral aneurysm, in order to prevent the coil in the cerebral aneurysm from being drawn out together with the secondary catheter. Moreover, when the tip of the secondary catheter is to be moved further inside in the cerebral aneurysm, the surgeon manipulates the delivery wire in a withdrawing direction while inserting the secondary catheter so that the coil protruding from the tip of the secondary catheter will retreat inside the secondary catheter. Such an operation is carried out by the surgeon manipulating the secondary catheter and another surgeon manipulating the delivery wire. Each surgeon holds the Y connector with his/her left hand and manipulates the secondary catheter or delivery wire with the fingertip of his/her right hand.

The usage of a master slave system is known in such treatments. For example, Patent Document 2 (Japanese Patent Laying-Open No. 2000-42116) discloses a medical equipment manipulation device that allows greater dexterity for the surgeon in manipulating medical devices such as a catheter or wire inserted in a patient's body. According to Patent Document 2, a surgeon at the master side manipulates a linear object taking the form of a tube or wire corresponding to a catheter or wire. The amount of advancing or rotational movement of the linear object is detected by means of a master actuator. A slave actuator for driving the catheter or wire to be inserted into the body is provided at the slave side. A master slave system is configured based on a master/slave control program executing position alignment type control.

Thus, treatment using a catheter requires skill. Moreover, the manipulation of the catheter or delivery wire requires critical control. For the purpose of improving the handling of the catheter and delivery wire in catheter medical treatment, a master slave apparatus as disclosed in Patent Document 2 (Japanese Patent Laying-Open No. 2000-42116) and Patent Document 3 (Japanese Patent Laying-Open No. 2001-157662) are proposed.

### PRIOR ART DOCUMENTS

### PATENT DOCUMENTS

- Patent Document 1:: Japanese National Patent Publication No. 8-506512
- Patent Document 2:: Japanese Patent Laying-Open No. 2000-42116
- Patent Document 3:: Japanese Patent Laying-Open No. 2001-157662

### SUMMARY OF THE INVENTION

### PROBLEMS TO BE SOLVED BY THE INVENTION

In the event of coil embolization medical treatment of a cerebral aneurysm by means of the device of Patent Document 1 (Japanese National Patent Publication No. 8-506512), the coil cannot be detached from the master side since the master slave apparatus is absent of a function to detach the coil for occlusion.

In the case of the electrical type, an electrode must be connected to the delivery wire. At the time of the cerebral aneurysm being filled with coil, the major part of the delivery wire will be located in the catheter. The exposed length of the delivery wire in such a state is small, and the relatively short exposed portion of the delivery wire is placed in the driving circuit of the slave. Therefore, the process of inserting an electrode from outside the slave for connection to the delivery wire at the site in proximity to the driving portion requires an additional step of opening, for example, the slave case, which is cumbersome.

This cumbersome step in connecting an electrode may disturb the surgical procedure such as unintentionally moving the delivery wire at the time of electrode connection.

Moreover, in view of the conventional procedure being carried out based on the cooperation between two surgeons, there may be a difference in the procedure or skill between the two, which may lead to longer time required for sufficient cooperation during the medical treatment, or stress on the surgeons.

Two issues identified in the system of Patent Document 2 (Japanese Patent Laying-Open No. 2000-42116), on which the preamble of claim 1 is based, will be described hereinafter.

The first issue is that the linear object corresponding to a catheter and wire must be manipulated by two surgeons. This is because the manipulation of a linear object set forth in Patent Document 2 is analogous to that in a conventional surgery.

The second issue lies in the scaling function to improve the operability in the master slave system. The scaling function is directed to modifying the ratio of the amount of advancing movement of the linear body at the slave side to the amount of operation at the master side for dexterous manipulation to achieve the scale suitable for hand operation. By setting the scaling value at N times, the amount of advancing movement at the slave side with respect to manipulation at the master side will be 1/N times, which is the reciprocal of the scaling value, whereas the corresponding length of the manipulation unit of the linear object at the master side will be N times that of the linear object at the slave side. Therefore, a manipulation unit having a length in proportion to the largest scale value may have to be prepared in the worst case. This is not practical since the catheter and wire has a length of one meter to two meters. Further, since the manipulated linear objects are overlapped concentrically, the inner side linear object must be manipulated at the area where the outer linear object is absent. This means that the distance between the two surgeons in the operation will become longer in proportion to the scaling value, causing more difficulty in the cooperation between the surgeons.

In the treatment using a master slave apparatus, not only the procedure of inserting a linear object such as guide wire, catheter, and a delivery wire into the body, but also the procedure of extracting the linear object from the body is carried out using the master slave apparatus. However, the master device of the master slave apparatus is produced based on the intention to allow the dexterous manipulation required during insertion of a linear object into a body. In other words, the master slave apparatus is designed such that the amount of advancing movement of the linear body is relatively smaller with respect to the amount of operation of the manipulation unit at the master device. Therefore, manipulating the manipulation unit of the master device to extract a linear object that is longer than or equal to 1 m from the body imposes a great burden on the operator.

### MEANS FOR SOLVING THE PROBLEMS

An object of the present invention is to provide a linear object manipulation control device that allows accurate manipulation of a linear object such as a catheter, delivery wire and guide wire inserted into a body, and that simplifies the operation.

In view of the above object, a linear object manipulation control device according to claim 1 includes a master having an externally operated manipulation unit, and a slave for applying a force in a longitudinal axial direction to a linear object inserted into a body based on an operation through the manipulation unit to move the linear object in the body.

The master includes an operation amount detection unit detecting an externally applied amount of operation of the manipulation unit, an operation amount transmission unit transmitting the amount of operation detected by the operation amount detection unit to the slave. The slave includes a linear object driving unit applying a force in the longitudinal axial direction to the linear object according to an applied driving signal, an operation amount reception unit receiving the amount of operation from the operation amount transmission unit, and a conversion unit converting the operation amount received by the operation amount reception unit into a driving signal for output to the linear body driving unit. The linear object includes a catheter and a wire inserted in the catheter. The manipulation unit includes a catheter manipulation unit and a wire unit disposed in close proximity at a surface of a casing of the master for manipulating individually a catheter and wire.

The master further includes a load applying unit applying a load according to a driving signal received from the slave to the manipulation unit. The conversion unit of the slave converts the amount of operation received from the operation amount reception unit into a driving signal for output to the linear object driving unit and the master.

Preferably, the master further includes a notification unit externally notifying a driving signal received from the slave.

Preferably, the master is portable.

Preferably, the master further includes an externally operated switching unit at the casing surface for switching the operation of the manipulation unit valid or invalid.

Preferably, each of the catheter manipulation unit and wire manipulation unit includes a rotator at the casing surface, rotationally operated arbitrarily from an external source. The linear object driving unit includes a roller rotating according to an applied driving signal. A force is applied in the longitudinal axial direction to the linear object in conjunction with the rotation of the roller. The level of the driving signal is proportional to the rotating amount of operation of the rotator.

Preferably, the communication is wired or wireless communication.

Preferably, the master further includes a storage unit storing the amount of operation and the level of a corresponding driving signal in association.

Preferably, the linear object is formed of a conductive material, and has one end side connected with a predetermined member to arrive at a target site in the body via a connecting portion formed of a material that can melt by an electric current.

The slave includes a power supply unit connected to a first electrode unit connected in advance to a body, a second electrode unit allowed to be connected outside the body to the other end side of the linear object in a state inserted in the body, and a control unit switching between establishing or not establishing a closed electrical circuit constituted of the first electrode unit, power supply unit, second electrode unit, linear object, and body, based on an externally applied instruction through operation of the manipulation unit by an external source.

Preferably, the slave further includes an electrode shifting unit for shifting the second electrode unit to form contact with the linear object in establishing the closed electrical circuit. The control unit establishes the closed electrical circuit after the second electrode unit forms contact with the linear object.

Preferably, the linear object has a fixed length. The slave further includes a position detection unit detecting the position of the other end of the linear object. The control unit does not establish the closed electrical circuit when the position detected by the position detection unit is outside a predetermined range when the instruction is applied.

Preferably, the master outputs the instruction to said slave when detection is made that the operation of the manipulation unit has been performed for a predetermined time.

Preferably, the manipulation unit includes a start manipulation unit operated to establish a closed electrical circuit. In the case where a closed electrical circuit is not to be established when detection is made that the instruction is applied through an operation of the start manipulation unit, the slave outputs an instruction of sound output to the master. The master outputs a predetermined sound in response to input of an instruction of sound output from the slave.

Preferably, the manipulation unit includes a start manipulation unit operated to establish a closed electrical circuit, and a stop manipulation unit operated to cut the establishment of the closed electrical circuit. The master outputs a different sound for each of the start manipulation unit and the stop manipulation unit when a relevant manipulation unit is operated.

Preferably, the manipulation unit includes a linear object manipulation unit operated to move the linear object. The slave further includes a linear object driving unit to apply a force in the longitudinal axial direction to the linear object to move the linear object in the body according to displacement in the amount of operation of the linear object manipulation unit applied from the master. During the period of establishment of the closed electrical circuit, the movement of the linear object by the linear object driving unit is stopped.

Preferably, the master maintains the amount of operation of the linear object manipulation unit at the amount immediately before establishment of the closed electrical circuit during the period of establishment of the closed electrical circuit.

Preferably, the linear object manipulation control device further includes an extraction unit for extracting the linear object from the body.

The extraction unit includes an actuator working to extract the linear object with a predetermined force when the manipulation unit is operated, reducing the extracting force of the linear object when an extracting speed of the linear object is greater than a predetermined speed, and stopping extraction of the linear object when an extracted length of the linear object arrives at a predetermined length.

According to the configuration, when the operator operates the manipulation unit, a linear object such as guide wire, catheter and delivery wire inserted in a body is automatically extracted by a predetermined force by the actuator. When the extracting speed of the linear object is greater than a predetermined speed, the extracting force of the linear object is reduced. Accordingly, slipping between a roller feeding out the linear object and the linear object can be prevented. Accordingly, the extracted length of the linear object can be calculated accurately from the number of rotations or the like of the roller to allow the linear object to be extracted accurately by just a predetermined length. Accordingly, the linear object can be extracted from the body safely and properly with an appropriate force. Thus, there can be provided a linear object manipulation control device that can alleviate the burden on the operator in extracting a linear object.

Preferably, there are a plurality of the above-described linear objects. The manipulation unit includes a plurality of extraction manipulation units. The actuator functions to extract a different linear object, among the plurality of extraction manipulations units, according to the extraction manipulation unit operated by the operator.

According to the present configuration, an arbitrary linear object among the guide wire, the catheter, and delivery wire can be extracted.

Preferably, the actuator works to extract a linear object by just a length differing according to the extraction manipulation unit operated by the operator, among the plurality of extraction manipulation units.

According to the present configuration, the extracting length of the linear object can be selected. Therefore, the work of extracting a linear object can be performed in a delicate manner.

Preferably, the extraction unit includes a feeding roller and a pressing roller for sandwiching a linear object. The actuator moves the linear object by rotating the feeding roller.

In accordance with the present configuration, the linear object can be moved by rotating the feeding roller that sandwiches the linear object with the pressing roller.

Preferably, the extraction unit further includes a resilient member supporting the pressing roller.

In accordance with the present configuration, the linear object can be sandwiched reliably between the feeding roller and pressing roller by the resilience of the resilient member.

Preferably, the extraction unit further includes a detector detecting the number of rotations of the feeding roller. The extracted length of the linear object is calculated based on the number of rotations of the feeding roller.

In accordance with the present configuration, the extracted length of the linear object can be calculated accurately from the number of rotations of the feeding roller.

Preferably, the actuator is a motor. The feeding roller is attached directly to the motor.

In accordance with the present configuration, the feeding roller that feeds out the linear object is directly attached to the motor without the intervention of a reducer or the like. Accordingly, mechanical loss can be reduced to allow the linear object to be moved efficiently.

Preferably, the extracting force of the linear object is determined according to the value of the current driving the motor.

In accordance with the present configuration, the extracting force of the linear object can be controlled by specifying the value of the current for driving the motor. Therefore, the extracting force of the linear object can be controlled accurately by taking advantage of feedback control or the like.

Preferably, the linear object is at least one of a catheter, delivery wire, and guide wire.

In accordance with the present configuration, at least one of a catheter, delivery wire, and guide wire can be extracted from the body safely and readily by an appropriate force. There are a plurality of linear objects. A plurality of manipulation units are provided. The actuator works to extract a different linear object according to the manipulation unit operated by the operator, among the plurality of manipulation units.

### EFFECTS OF THE INVENTION

According to the present invention, the catheter manipulation unit and wire manipulation unit are arranged in close proximity at the surface of the casing of the master. Therefore, the catheter manipulation unit and wire manipulation unit can be operated by one person. Therefore, the movement of the catheter and wire can be manipulated by remote control through one person. As a result, the cooperation in operation between two surgeons, as in prior art, is no longer required. The time for the surgical procedure in association with catheter manipulation can be reduced, and the stress on the surgeon related to cooperation can be reduced.

The present invention allows a predetermined member to be detached from a linear member at a target site through remote control operation from the master side. The cumbersome operation of externally connecting a second electrode unit to the linear object can be eliminated. Accordingly, the possibility of the linear object being shifted at the time of connecting the second electrode unit is eliminated.

### BRIEF DESCRIPTION OF THE DRAWINGS

Fig. 1 represents an overall schematic configuration of an embolization coil detachment device that is an example of a linear object manipulation control device according to a first example.
Fig. 2 represents an example of an operation panel provided at the master in the present embodiment.
Fig. 3 is a diagram to describe a specific configuration of a slave according to the first embodiment.
Fig. 4 represents a schematic configuration of hardware of a master control unit according to the first embodiment.
Fig. 5 represents a schematic configuration of hardware of a slave control unit according to the first embodiment.
Fig. 6 represents a functional configuration of the master control unit according to the first embodiment.
Fig. 7 represents a functional configuration of the slave control unit according to the first embodiment.
Fig. 8 represents an example of a mechanism to transmit the amount of operation of a delivery wire drive manipulation unit according to the first embodiment.
Fig. 9 represents a mechanism for a fixed position control unit according to the first embodiment.
Fig. 10 is a flowchart of the process for detaching an embolization coil according to the first embodiment.
Fig. 11 represents an overall schematic configuration of a manipulation device for a medical linear object that is an example of a linear object manipulation control device according to an embodiment of the invention.
Fig. 12 represents the transmission and reception of signals between the slave and master, and associated sections of the master according to the second embodiment.
Fig. 13 represents transmission and reception of signals between the slave and master, and associated sections of the slave according to the second embodiment.
Fig. 14 represents an appearance of the master according to the second embodiment.
Fig. 15 schematically represents a drive mechanism of a catheter and delivery wire at the slave side according to the second embodiment.
Fig. 16 schematically represents a circuit configuration to apply a rotation load to a secondary catheter manipulation unit according to the second embodiment.
Fig. 17 schematically represents a circuit configuration to apply a rotation load to a delivery wire manipulation unit according to the second embodiment.
Fig. 18 represents another configuration of a master according to the second embodiment.
Fig. 19 represents a casing of the master of Fig. 18, wherein (A) is a top view of the casing and (B) shows an internal configuration in the casing.
Fig. 20 represents a schematic configuration of an extraction device of a linear object according to a second embodiment.
Fig. 21 is a perspective view of a delivery wire driving unit according to a third embodiment.
Fig. 22 is a sectional view of a delivery wire driving unit according to a third embodiment.
Fig. 23 represents another schematic configuration of an extraction device of a linear object according to the third embodiment.
Fig. 24 represents a switch and the like in the control circuit according to the third embodiment.
Fig. 25 is a diagram to describe an inserting operation of a coil in a conventional coil embolization.

### MODES FOR CARRYING OUT THE INVENTION

### (First Example)

An example will be described hereinafter with reference to the drawing. In the following, the same elements have the same reference characters allotted. Their designation and function are also identical. Therefore, detailed description thereof will not be repeated.

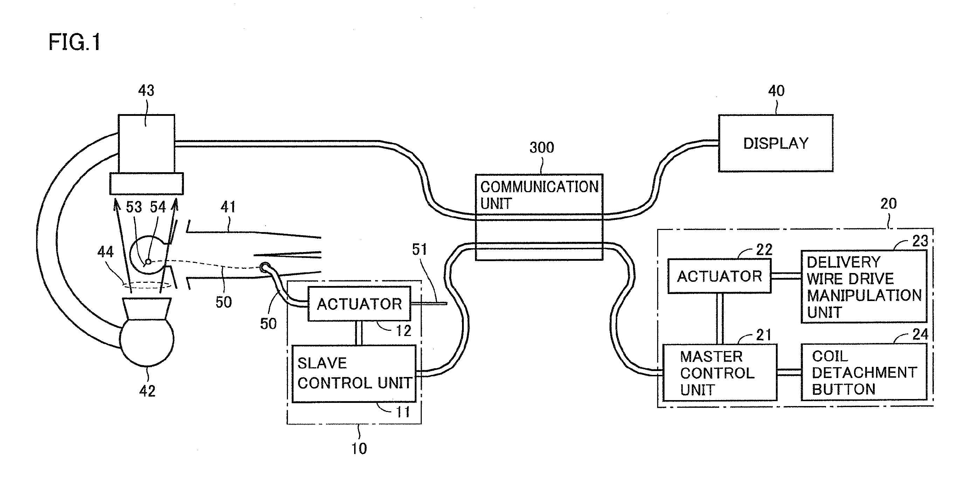

Fig. 1 represents an overall schematic configuration of an embolization coil detachment device that is an example of a linear object manipulation control device according to the present embodiment. The embolization coil detachment device includes a slave 10, a master 20 installed distant from slave 10, and a communication unit 300 for connection between slave 10 and master 20 through communication. Slave 10 and master 20 communicate by a relay of communication unit 300 through a cable. Although wired communication through a cable is employed here in view of the possibility of erroneous operation of other instruments provided in the neighborhood, wireless communication may be employed in the case where there is no such restriction.

The embolization coil detachment device further includes an X-ray radiation device 42 and an X-ray imaging device 43 for applying an X-ray 44 to the involved region of a patient 41 into which a delivery wire 51 and a catheter 50 are inserted to visualize the region of interest, and also a display 40 to receive image signals output from X-ray imaging device 43 via communication unit 300 and provide an image display to an external source.

The user operating at master 20 side can operate a delivery wire drive manipulation unit 23 while confirming the movement of delivery wire 51 through an X-ray image by X-ray imaging device 43 on the screen of display 40. Delivery wire 51 can be identified as an image since it is formed of an X-ray non-transmitting material. Catheter 50 will not appear on the X-ray image since it is made of resin. However, the position of catheter 50 can be identified since delivery wire 51, when present inside, appears on the X-ray image. In the image of delivery wire 51, a marker made of metal that does not transmit X-ray is attached at the tip or partway of catheter 50. The position of the tip of catheter 50 can be confirmed by identifying the location of the marker appearing in the X-ray image.

Delivery wire 51 is employed to place a coil 53, which is made of platinum, for embolization within the cerebral aneurysm that is the target site. Coil 53 formed of platinum is connected to the tip of delivery wire 51. A connecting portion 54 between coil 53 and delivery wire 51 is formed of a material such as stainless steel that melts readily in blood by electric current. As used herein, melting by electric current refers to the metal of connecting portion 54 melting by conducting an electric current.

Slave 10 includes an actuator 12 that is a driving mechanism for inserting/extracting catheter 50 and delivery wire 51 into/from the body of patient 41, and a slave control unit 11 for controlling the operation of actuator 12.

Master 20 includes delivery wire drive manipulation unit 23 operated by a surgeon to insert/extract delivery wire 51, an actuator 22 receiving an operation signal of delivery wire drive manipulation unit 23 to derive a driving signal to drive delivery wire 51 according to the input operation signal, a master control unit 21, as well as a coil detachment button 24.

Coil detachment button 24 is operated to detach coil 53 connected at the tip of delivery wire 51 from delivery wire 51. Master control unit 21 controls each element in master 20.

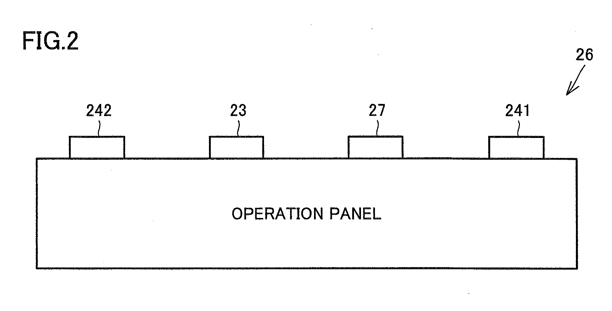

Fig. 2 represents an example of an operation panel 26 provided at master 20. Operation panel 26 includes a coil detachment start button 241 and a coil detachment stop button 242 corresponding to coil detachment button 24, delivery wire drive manipulation unit 23, and an end button 27 operated to designate the ending of a series of operations.

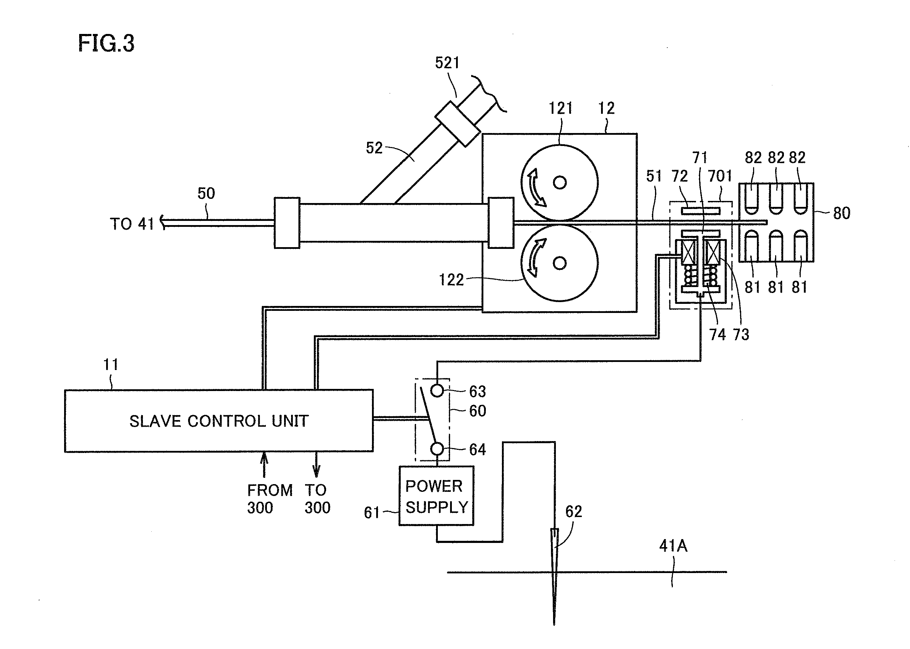

An example of a specific configuration of slave 10 will be described with reference to Fig. 3. In Fig. 3, actuator 12, a conduction switch 60, a position detection unit 80 to detect the position of delivery wire 51, and a movable electrode unit 701 are connected to slave control unit 11. A Y connector 52 is provided at the region where actuator 12 is connected with catheter 50. Movable electrode unit 701 and position detection unit 80 are provided in association with delivery wire 51 that is inserted/extracted into/out from the body of patient 41 through actuator 12.

Delivery wire 51 can be inserted from the actuator 12 side input port of Y connector 52, and a pharmacological agent can be introduced from the other input port 521 of Y connector 52. For example, physiological saline can be introduced from the other input port 521 to reduce friction between catheter 50 and delivery wire 51. An angiography agent can be introduced from the other input port 521 after catheter 50 inserted in the blood vessel is guided to the target site from outside the body to allow the angiography agent to arrive at the target site in the body.

Actuator 12 functions as a delivery wire driving mechanism to apply a force in the longitudinal axial direction to delivery wire 51 for movement in order to insert and extract delivery wire 51 into and out from patient 41 via Y connector 52. Specifically, actuator 12 includes a pressing roller 121 and a feeder 122 provided relatively to sandwich delivery wire 51. Feeding roller 122 is driven by a motor not shown to rotate in the direction of the arrow. The amount of rotation and rotating direction of the motor are determined depending upon the displacement of the applied amount of operation of delivery wire drive manipulation unit 23 (the difference between the immediately preceding amount of operation and the current amount of operation). Feeding roller 121 rotates in conjunction with the rotation of the motor, whereby delivery wire 51 placed on the rotating plane (roller plane) advances in the inserting/extracting direction in conjunction with the rotation of feeding roller 122. Pressing roller 121 functions to push delivery wire 51 against the rotating face of feeding roller 122 while rotating, in conjunction with the rotation of feeding roller 122 in the direction of the arrow.

Movable electrode unit 701 is provided at the side of actuator 12 opposite to Y connector 52. Movable electrode unit 701 includes electrodes 71 and 72 provided facing each other with delivery wire 51 extending from actuator 12 therebetween, a solenoid 73 and a spring 74 provided in association with electrode 71. Electrode 71 is movable by solenoid 73 of movable electrode unit 701.

When electrode 71 is located at the position shown in Fig. 3, i.e. when current is not supplied from slave control unit 11 to solenoid 73 and electrode 71 does not form contact with delivery wire 51, the movement of delivery wire 51 is not impeded.

Position detection unit 80 is provided at the side of movable electrode unit 701 opposite to actuator 12. Position detection unit 80 includes a row of a plurality of photosensors 81 that are light receiving elements (hereinafter, referred to as "photo line sensor", and a row of a plurality of light sources 82 that are light emitting elements, each row formed opposite to each other with delivery wire 51 extending from movable electrode unit 701 therebetween.

Light source 82 is formed of, for example, an LED (Light Emitting Diode). Photosensors 81 are provided corresponding to light sources 82, respectively. Each photosensor 81 is provided at a position that can receive light emitted from a corresponding light source 82 to output a light receiving signal of a level according to the received light.

Plurality of light sources 82 are aligned in a row, parallel to delivery wire 51. Similarly, a plurality of photosensors 81 are aligned in a row, parallel to delivery wire 51. Therefore, when the emitted light from light source 82 is blocked by delivery wire 51, the level of the light receiving signal at a corresponding photosensor 81 is reduced. By detecting the position of a photosensor 81 in the row of photo line sensors having a light receiving signal lower in level than a predetermined level, the position of delivery wire 51 is detected.

Conduction switch 60 is an open/close switch including terminals 63 and 64. Terminal 64 is connected to a power supply 61. Electrode 71 of movable electrode unit 701 is connected to terminal 63. The opening/closing operation of conduction switch 60 is under control of slave control unit 11. A syringe needle 62 that is electrically conductive to allow detachment of coil 53 is pierced into a body 41 A of patient 41. Syringe needle 62 is connected to power supply 61 through a conductive wire. When conduction switch 60 is ON (closed), shorting occurs between terminals 63 and 64 to attain a conducting state.

When conduction switch 60 is ON (closed) for a predetermined period of time, a current is supplied to the coil of solenoid 73 in movable electrode unit 701 through conduction switch 60. Spring 74 contracts by the generated electromagnetic force, whereby electrode 71 connected integrally with spring 74 moves towards delivery wire 51 to form contact. When electrical conduction is terminated (conduction switch 60 is turned OFF from an ON state), spring 74 returns to its former state.

Accordingly a closed electrical circuit of "movable electrode unit 701 → delivery wire 51 → connecting portion 54 → coil 53 → body 41A → syringe needle 62 → movable electrode unit 701" is established. Connecting portion 54 is electrically melted by an electrical signal from power supply 61, whereby coil 53 is detached from delivery wire 51. Thus, the ON state of conduction switch 60 for a predetermined period of time allows coil 53 to be detached from delivery wire 51.

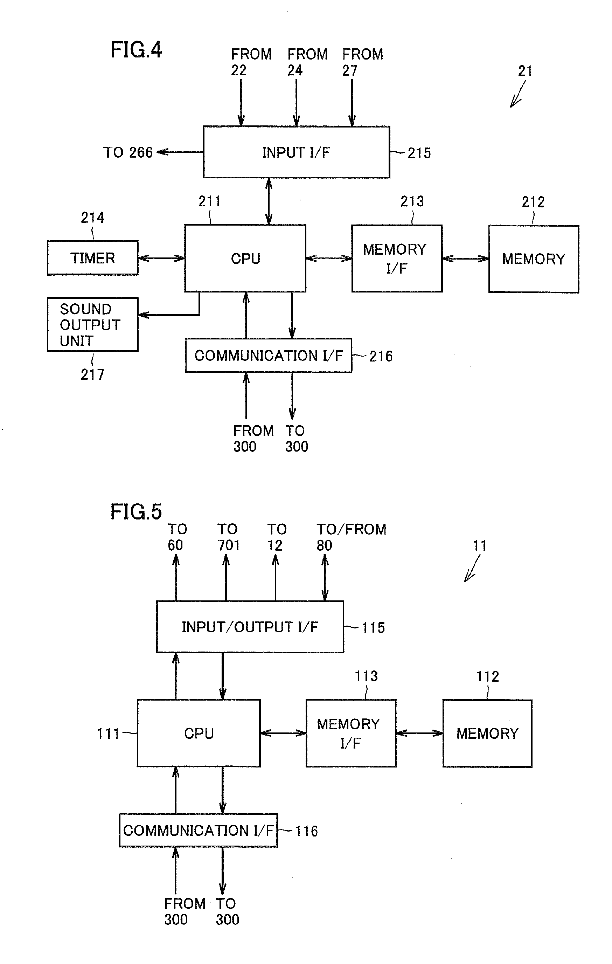

Fig. 4 represents a schematic configuration of the hardware of master control unit 21. Master control unit 21 includes a CPU (Central Processing Unit) 211, a memory I/F (Interface) 213, a memory 212 accessed by CPU 211 via a memory I/F 213, a timer 214, an input I/F 215, a communication I/F 216 for connecting communication unit 300 with CPU 211, and a sound output unit 217 to output sound outside. Input I/F 215 receives a driving signal output from actuator 22 and an operation signal of coil detachment button 24 and end button 27, which is applied to CPU 211. CPU 211 controls and monitors other elements in master control unit 21 in a centralized manner.

Fig. 5 shows a schematic configuration of the hardware of slave control unit 11. Slave control unit 11 includes a CPU 111, a memory I/F 113, a memory 112 that is accessed by CPU 111 through memory I/F 113, an input/output I/F 115, and a communication I/F 116 for connecting communication unit 300 with CPU 111.

CPU 111 control and monitors each other element in slave control unit 11 in a centralized manner. Input/output I/F 115 outputs a control signal from CPU 111 to power switch 60 and actuator 12. The sensor signal from each photosensor 81 in position detection unit 80 is output to CPU 111, and a light emission instruction signal output from CPU 111, designating light emission, is provided to a relevant light source 82.

The sensor signal output from each photosensor 81 includes a light receiving signal indicating the amount of light received from a corresponding light source 82, and a row position signal indicating the position of photosensor 81 in a row of photo line sensors.

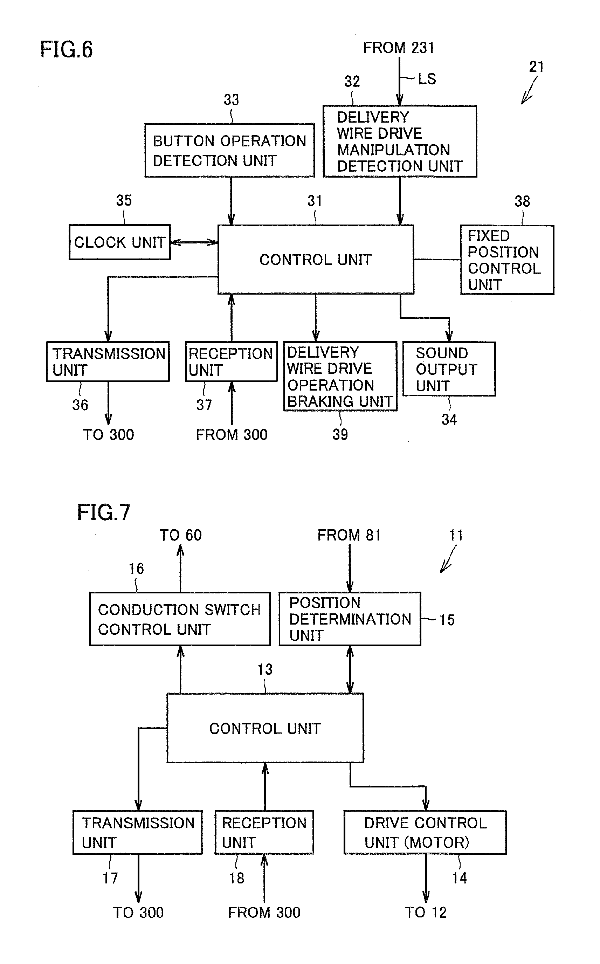

A functional configuration of master control unit 21 will be described with reference to Fig. 6. Master control unit 21 includes a control unit 31 corresponding to CPU 211, a delivery wire drive manipulation detection unit 32, a button operation detection unit 33, a sound output unit 34 corresponding to sound output unit 217, a clock unit 35 corresponding to timer 214, a transmission unit 36 and a reception unit 37 corresponding to communication I/F 216, a fixed position control unit 38, and a delivery wire drive operation braking unit 39. Fixed position control unit 38 and delivery wire drive operation braking unit 39 will be described afterwards. Each element in Fig. 6 is a function implemented by a program or hardware, or a combination thereof.

Referring to Fig. 7, slave control unit 11 includes a control unit 13 corresponding to CPU 111, a drive control unit 14 for controlling the drive of rollers 121 and 122 in actuator 12, a position determination unit 15 provided corresponding to position detection unit 80, a conduction switch control unit 16 provided corresponding to conduction switch 60, and a transmission unit 17 and a reception unit 18 provided corresponding to communication I/F 116. Each component and element in Fig. 7 can be realized by a program, hardware, or a combination thereof.

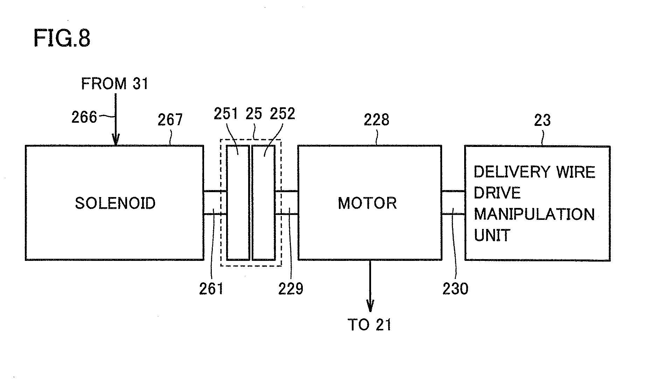

Fig. 8 shows an example of a mechanism to transmit the amount of operation of delivery wire drive manipulation unit 23. A motor 228, a clutch 25, and a solenoid 267 are provided in association with delivery wire drive manipulation unit 23. Delivery wire drive operation braking unit 39 includes a clutch 25 and a solenoid 267. Motor 228 includes actuator 22.

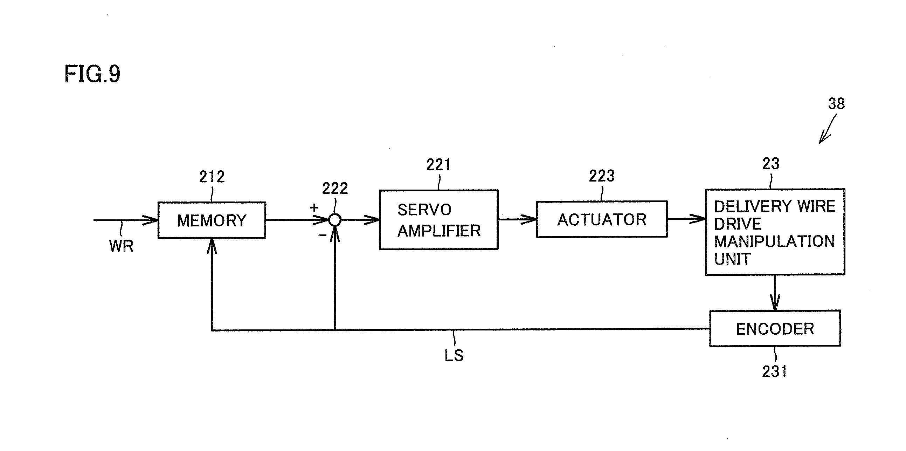

Referring to Fig. 8, delivery wire drive manipulation unit 23 is fixed to the casing of the body of operation panel 26, arbitrarily rotated in response to an external operation, for example. The rotating amount of operation (rotating direction, rotating amount (angle)) is transmitted to motor 228 via a rotary shaft 230. Motor 228 rotates according to the transmitted amount of operation. A signal indicating the rotation of motor 228 (rotating direction and rotation amount) is detected by an encoder 231 (refer to Fig. 9) provided in association with motor 228 to be output to master control unit 21 as a driving signal. Further, a clutch 25 and a solenoid 267 are provided in association with motor 228. Clutch 25 is based on a lapping configuration of friction plates 251 and 252. A motor shaft 229 is connected to friction plate 252. A shaft 261 on the part of solenoid 267 is connected to friction plate 251.

Solenoid 267 is employed for controlling clutch 25. When a control signal 266 is applied to solenoid 267, motor 228 is mechanically locked. Control signal 266 is applied for a predetermined period of time establishing a closed electrical circuit with conduction switch 60 in an ON state. Accordingly, the rotation of movable delivery wire drive manipulation unit 23 is disabled over a predetermined period of time. Motor 228 will not be locked mechanically during a period of control signal 266 not being applied to solenoid 267.

Upon energization of solenoid 267 by an applied control signal 266, shaft 261 extends towards clutch 25. Therefore, friction plate 251 connected to shaft 261 is brought into contact with friction plate 252 connected to motor shaft 229. The two friction plates brought into contact with each other attain a friction engaged state, whereby motor shaft 229 is forced to stop rotation. Thus, motor 228 is mechanically locked. When motor 228 attains a locked state, delivery wire drive manipulation unit 23 attains a state in which operation is not allowed.

The friction engagement is effected in a direction at right angles to the movable direction of delivery wire drive manipulation unit 23 to prevent offset of delivery wire drive manipulation unit 23 by the friction engagement. An electromagnetic clutch or the like may be employed instead of electromagnetic solenoid 267.

Fig. 9 represents a mechanism for fixed position control unit 38. Fixed position control unit 38 includes an actuator 223 and an encoder 231 corresponding to motor 228, and a servo amplifier 221 for servo control, a difference detector 222, and a memory 212.

Upon operation of coil detachment start button 241, coil 53 is detached from connecting portion 54 to place coil 53 at the involved region (cerebral aneurysm). In order to place coil 53 accurately at the involved region (cerebral aneurysm), any movement of delivery wire 51 is disabled by means of the mechanism of Fig. 9 during a predetermined time where conduction switch 60 is ON.

According to the mechanism of Fig. 9, when coil detachment start button 241 is operated, a write request WR is applied from CPU 211 (control unit 31) to memory 212 via memory I/F 213. Memory 212 responds to a write request WR to store a position signal LS currently output from encoder 231 as data. Encoder 231 detects the amount of operation of delivery wire drive manipulation unit 23 as the amount and direction of rotation of motor 228, which is converted into position signal LS for output.

Subsequently, a position signal LS corresponding to operation of coil detachment start button 241 indicated by the data read out from memory 212, and position signal LS currently detected by encoder 231 are applied to difference detector 222. Difference detector 222 detects the difference in level between the two applied position signals LS to output the detected difference signal to servo amplifier 221. Servo amplifier 221 applies a current signal of a level corresponding to the applied difference to actuator 223. Therefore, actuator 223 rotates delivery wire drive manipulation unit 23 by just an amount corresponding to the difference. By such servo control through a closed loop, the position of delivery wire drive manipulation unit 23 is controlled to be maintained at the position immediately preceding the operation of coil detachment start button 241. Therefore, the position of delivery wire 51 can be left at the position immediately preceding manipulation of coil detachment start button 241.

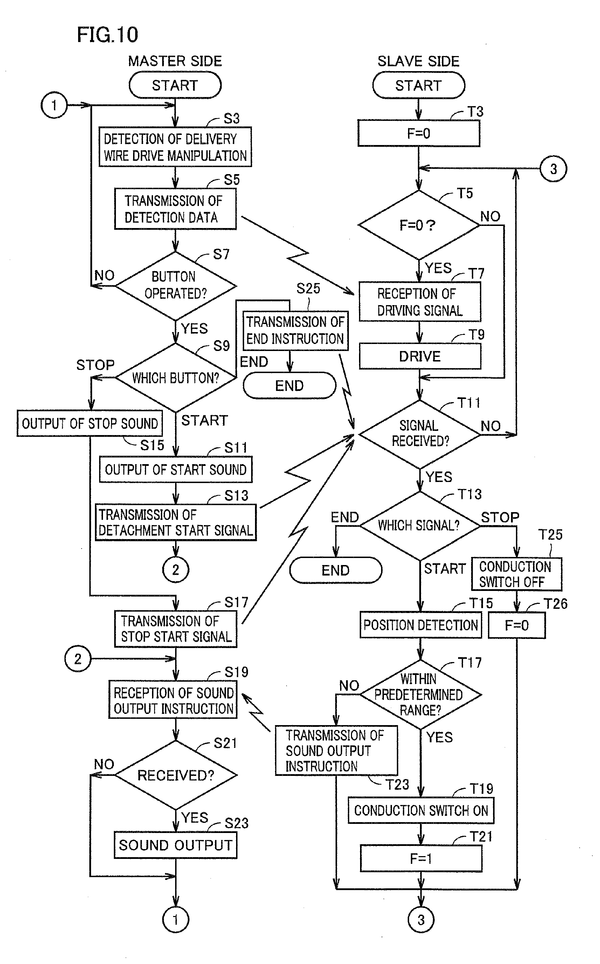

Referring to the flowchart in Fig. 10, the procedure for embolization coil detachment according to the present embodiment will be described. A program according to the flowchart is prestored in memories 112 and 212. The process of embolization coil detachment is implemented by CPUs 111 and 211 reading out each instruction code in the relevant program from the memory and executing each instruction code.

First, on the part of master 20, delivery wire drive manipulation detection unit 32 detects the amount of operation of delivery wire drive manipulation unit 23 based on a driving signal indicated by position signal LS by an operation of delivery wire drive manipulation unit 23 (step S3).

The detected amount of operation data is transmitted towards slave 10 via transmission unit 36 by control unit 31 (step S5). At slave 10, actuator 12 is driven according to the received amount of operation data, whereby delivery wire 51 or catheter 50 is moved to be inserted/extracted into/out from body 41A.

On the part of master 20, button operation detection unit 33 detects whether any button on operation panel 26 has been operated or not (step S7). When a button operation is not detected, control proceeds to step S3.

The surgeon operating delivery wire drive manipulation unit 23 can operate delivery wire drive manipulation unit 23 while confirming the current advancing state of delivery wire 51 through the screen displayed at display 40 by X-ray imaging device 43. Further, the state of the cerebral aneurysm being filled with coil 53 can also be visualized. Therefore, the surgeon confirming the displayed screen on display 40 operates coil detachment start button 241 when the timing to detach coil 53 is determined. Upon confirming detachment through the display screen, coil detachment stop button 242 is operated to stop the detachment operation. Further, end button 27 is operated after delivery wire 51 is drawn out from patient 41 and determination is made that the operation for the embolization of the cerebral aneurysm by coil 53 has ended.

When detection is made of an operation of any of the buttons set forth above (YES at step S7), button operation detection unit 33 detects the type of the operated button and provides the detected type to control unit 31 (step S9). Since each button on operation panel 26 outputs a signal of a unique level in response to the operation, the type of the button operated can be detected by looking into a table in which the type of a button is registered in advance corresponding to a signal level.

When detection is made that the detected type indicates "stop" corresponding to operation of coil detachment stop button 242 ("stop" at step S9), control unit 31 instructs sound output unit 34 to output a predetermined sound notifying "stop". Accordingly, a sound notifying "stop" is output from sound output unit 34 (step S15). Then, control proceeds to step S 17.

When detection is made that the detected type indicates "start" corresponding to the operation of coil detachment start button 241 ("start" at step S9), control unit 31 instructs sound output unit 34 to output a predetermined sound notifying "start". Accordingly, a sound notifying "start" is output from sound output unit 34 (step S 11). Then, control unit 31 transmits a detachment start signal towards slave 11 via transmission unit 36 and communication unit 300 (step S 13). Then, control proceeds to step S 19 that will be described afterwards.

When detection is made that the detected type indicates "end" corresponding to operation of end button 27 ("end" at step S9), control unit 31 transmits an end instruction signal towards slave 11 via transmission unit 36 and communication unit 300 (step S25). Then, the process on the part of master 20 ends.

At step S 17, control unit 31 transmits a start-of-stop signal towards slave 10 via transmission unit 36 and communication unit 300. Then, control proceeds to the process of step S 19.

At step S 19, control unit 31 detects whether a sound output instruction has been received from slave 10 via reception unit 37 (steps S19, S21). When reception is detected, control unit 31 causes sound output unit 34 to output a predetermined sound (step S23). Then, control proceeds to step S3.

Since the sound output instruction received at step S 19 is output when detection is made that the placement of delivery wire 51 is within a predetermined range at slave 10, the surgeon or the like on part of master 20 can confirm that delivery wire 51 is located within a predetermined range according to the sound output at step S23.

When detection is made that the sound output instruction is not received (NO at step S21), control returns to step S3 to perform the subsequent processes in a similar manner.

Since the sound differs between starting and stopping detachment, the user can confirm whether a button is erroneously operated or not in the operation of coil detachment start button 241 and coil detachment stop button 242.

The operation on part of slave 10 will be described hereinafter. On the part of slave 10, when the power or the like is turned ON to initiate the process, control unit 13 sets a control variable F temporarily to value 0 (step T3). Then, control unit 13 determines whether the value of variable F is 0 or not (step T5). As used herein, variable F is referred to in order to identify whether conduction switch 60 is turned ON/OFF. When determination is made that variable F does not designate 0 (NO at step T5), control proceeds to step T11 that will be described afterwards.

In contrast, when determination is made that variable F is 0 (YES at step T5), the drive signal transmitted from step S5 in the processing on part of master 20 is to be received (step T7). Drive control unit 14 drives and controls the rotation of feeding roller 122 in actuator 12 to insert/ extract delivery wire 51 based on the received driving signal (step T9).

Then, control unit 13 detects whether a signal transmitted from master 20 via reception unit 18 is received or not (step T11). When there is no reception of a signal, control proceeds to step T5 to carry out the subsequent processing in a similar manner. At step T11, a signal transmitted by any of steps S13, S17 and S25 is detected.

When detection is made of receiving a signal (step T11), control unit 13 analyzes the received signal to determine the type thereof (step T13). When determination is made that the received signal designates "end instruction" ("end" at step T13), the series of processing ends.

In contrast, when determination is made that the received signal designates "start instruction", control unit 13 instructs position determination unit 15 to detect the position of delivery wire 51. Position determination unit 15 responds to an input of the instruction to detect the position using position detection unit 80 (step T15). Determination is made whether the detected position designates a position in a predetermined range (step T17). Specifically, position determination unit 15 outputs a light emittance instruction signal to position detection unit 80 via input/output I/F 115. Each light source 82 effects light emittance based on the applied light emittance instruction signal. Emitted light is detected by a corresponding photosensor 81, from which a sensor signal is output.

Data indicating a predetermined range is prestored for position determination unit 15 to allow position determination unit 15 to compare the position data indicated by the row position signal of each input sensor signal and the data indicating the predetermined range to determine whether delivery wire 51 is located in a predetermined range based on the comparison result. The comparison result is output to control unit 13.

This determination will be described hereinafter. It is assumed that delivery wire 51 has one end connected to coil 53 and the other end located in position detection unit 80 in the state where delivery wire 51 is inserted in body 41A. Position determination unit 15 determines the position of the other end in position detection unit 80 corresponding to the time to detach coil 53 from delivery wire 51.

Although delivery wire 51 has a fixed length, the length differs depending upon the type. The position of the other end of delivery wire 51 located in position detection unit 80 corresponding to the timing of detaching coil 53 from delivery wire 51 will vary depending upon the length (type) of catheter 50. The data indicating a predetermined range according to the length of catheter 50 is prestored in memory 12. Position determination unit 15 reads out from memory 12 the data of the predetermined range according to the length of catheter 50 employed. The length of catheter 50 employed is provided in advance by an externally applied input. The predetermined range data is indicated by the row position of photosensor 81 that should succeed in receiving light among the photo line sensors.

Therefore, when detection is made that the light reception signal of photosensor 81 providing the row position signal indicated by the predetermined range data designates ON (succeeds in light reception), based on an input sensor signal, determination is made that delivery wire 51 is located within the predetermined range.

When detection is made by control unit 13 that the current position of delivery wire 51 is not within the predetermined range based on the determination result by position determination unit 15 (NO at step T17), a sound output instruction is transmitted towards master 20 via transmission unit 17 and communication unit 300 (step T23). Then, control proceeds to step T5.

Accordingly, when delivery wire 51 is not placed in a predetermined range, i.e. coil 53 has not yet arrived at the target site where coil 53 is to be detached from delivery wire 51, coil 53 will not be detached even if coil detachment start button 241 is operated. This is notified accordingly through sound on part of master 20 for the surgeon (refer to step S23). Thus, an erroneous operation of coil detachment start button 241 can be avoided.

In contrast, when detection is made that the current position of delivery wire 51 is within the predetermined range (YES at step T17), conduction switch control unit 16 is instructed to turn conduction switch 60 ON. In response to this instruction, slave control unit 11 initiates supply of current to solenoid 73 of movable electrode unit 701, whereby electrode 71 is shifted to form contact with delivery wire 51. Conduction switch control unit 16 maintains the ON state of conduction switch 60 for a predetermined period of time (step T19). Accordingly, connecting portion 54 is electrically melted to cause coil 53 to be detached from connecting portion 54. The period of time of supplying current to solenoid 73 and the ON duration must be sufficient to allow connecting portion 54 to be electrically melted.

When conduction switch 60 attains an ON state, control unit 13 sets the value of variable F at 1 (step T21). Then, control proceeds to step T3.

When detection is made that received signal designates "stop instruction" ("stop" at step T13), control unit 13 turns conduction switch 60 OFF via conduction switch control unit 16, and stops supply of current to solenoid 73 of movable electrode unit 701 (step T25). Accordingly, establishment of the closed electrical circuit is disrupted, and application of current to delivery wire 51 ends. Then, control unit 13 sets variable F at 0 (step T26). Then, control proceeds to step T5.

By operating coil detachment stop button 242 as set forth above, the detachment can be forced to stop in the event of erroneously operating coil detachment start button 241 or in the event of coil 53 being moved during detachment.

The detachment start button or detachment stop button on part of the master may be configured to work only when the button is operated over a predetermined period of time clocked by a clock 35 to avoid erroneous operation.

To prevent movement of catheter 50 and coil 53 during coil detachment, slave 10 may be configured to neglect an operation signal (driving signal) of catheter 50 and coil 53 from master 20 even when coil 53 or catheter 50 is operated on part of master 20. In addition, the device may be configured to disable operation of delivery wire drive manipulation unit 23 by effecting fixed positioning control to a fixed point (the position to start detachment) at delivery wire drive manipulation unit 23 on part of master 20, as shown in Fig. 9.

Alternatively, the manipulation unit on part of master 20 may be mechanically locked to be immobile during coil detachment, as shown in Fig. 8.

### (First Embodiment)

The present embodiment of the invention is directed to a linear object manipulation control device to allow manipulation of a catheter and delivery wire by one person.

The present embodiment will be described hereinafter with reference to the drawings. In the following, the same element has the same reference character allotted. Their designation and function are also identical. Therefore, detailed description thereof will not be repeated.

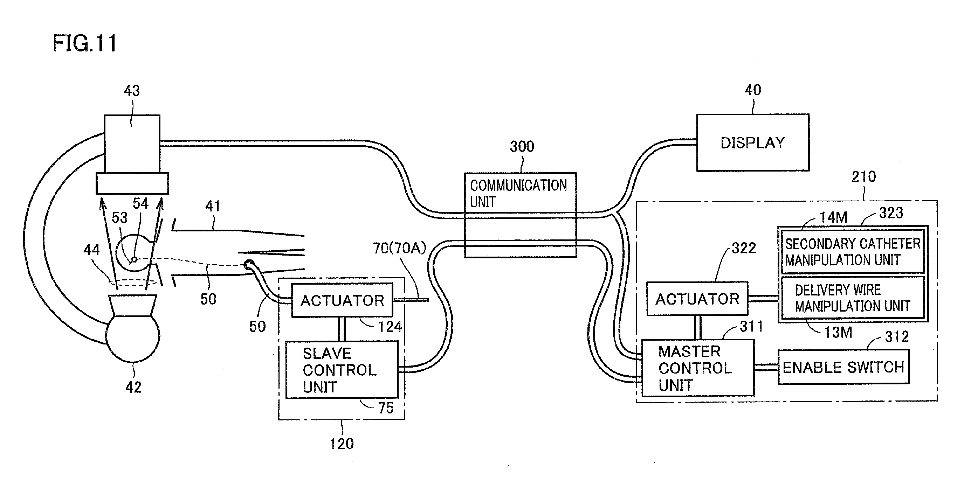

Fig. 11 represents an overall schematic configuration of a manipulation device for a medical linear object that is an example of a linear object manipulation control device according to the present embodiment. The medical linear object manipulation device includes a slave 120, a master 210 installed distant from slave 120, and communication unit 300 for connecting slave 120 and master 210 through communication. Slave 120 and master 210 communicate through relay by communication unit 300 via a cable. The communication path provided between slave 120 and master 210 is not limited to a wired communication as shown in Fig. 11, and may be a wireless communication.

The manipulation device for a medical linear object includes X-ray imaging device 43 and X-ray radiation device 42 to irradiate the involved region of patient 41 into which a delivery wire 70 and catheter 50 are inserted into the body with X-ray 44, and also display 40 receiving image signals output by X-ray imaging device 43 to provide an image to an external source on receiving the image signals through communication unit 300.

Slave 120 includes an actuator 124 that is a driving mechanism to insert/extract catheter 50 and delivery wire 70 into/out from the body of patient 41, and a slave control unit 75 for controlling the operation of actuator 124.

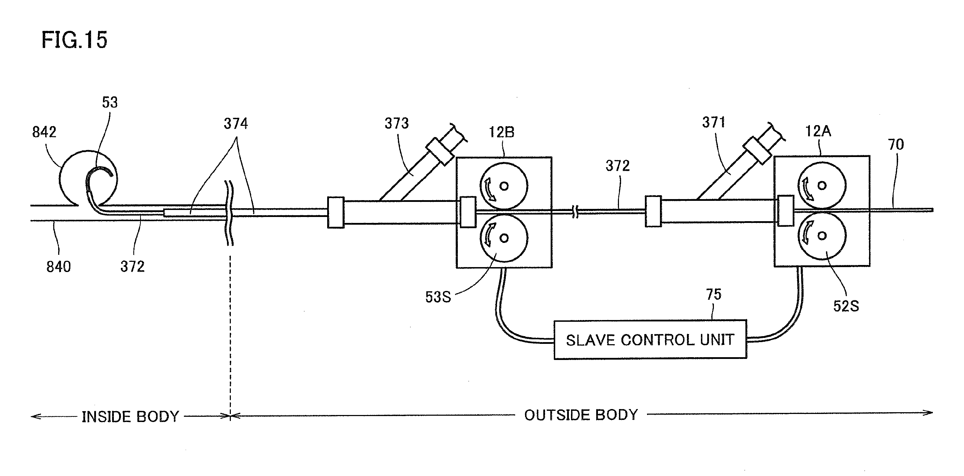

Master 210 includes a manipulation unit 323 operated by a surgeon to insert/extract delivery wire 70, an actuator 322 for applying a load on an external operation in association with manipulation unit 323, a master control unit 311 controlling each element in master 210, and an enable switch 312. Manipulation unit 323 includes a secondary catheter manipulation unit 14M for manipulating a second catheter 372 (refer to Fig. 15), and a delivery wire manipulation unit 13M for manipulating delivery wire 70. Enable switch 312 is operated to render the operation of manipulation unit 323 valid.

Likewise with the first embodiment, the surgeon can operate manipulation unit 323 while confirming the movement of delivery wire 70 through a screen on display 40. Delivery wire 70 can be visualized through an image since it is formed of an X-ray non-transmitting material. Further, likewise with the first embodiment, the surgeon can confirm the position of the tip of catheter 50 by identifying the location of the marker at the tip of catheter 50 appearing on the X-ray image.

A guide wire 70A is used instead of delivery wire 70 to guide catheter 50 formed of a soft resin material to a target site, for example, to a cerebral aneurysm. Guide wire 70A is formed of an X-ray non-transmitting material having a rigidity higher than that of catheter 50. The tip of guide wire 70A is flexible to prevent damaging the vessel. Guide wire 70A as well as catheter 50 with guide wire 70A passing therethrough can be made to advance so as to guide catheter 50 to the target site.

Delivery wire 70 is used to place coil 53 formed of platinum for embolization within a cerebral aneurysm that is the target site. Coil 53 is connected to the tip of delivery wire 70. Coil 53 and delivery wire 70 are connected by a connecting portion 54.

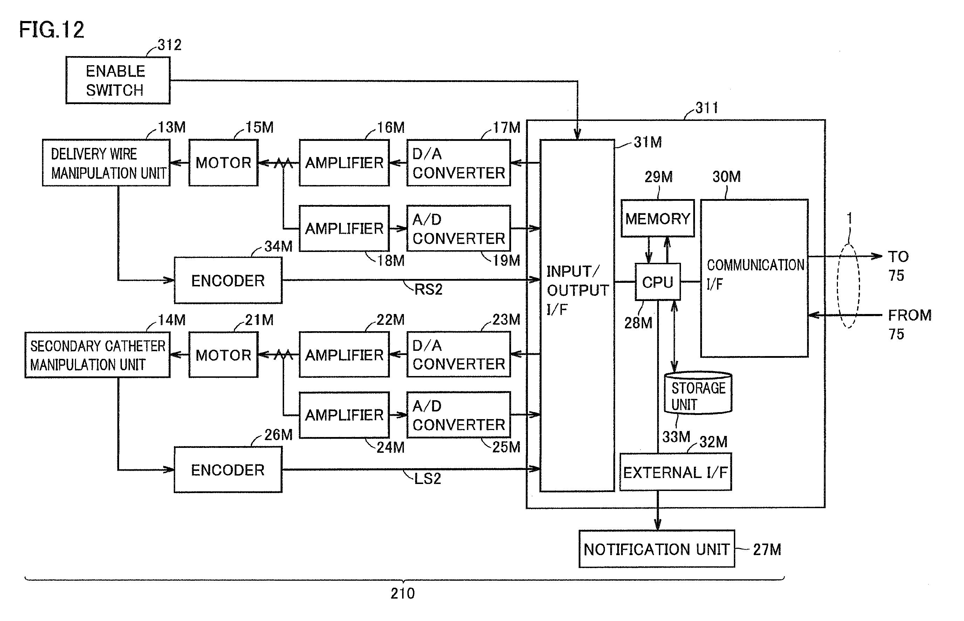

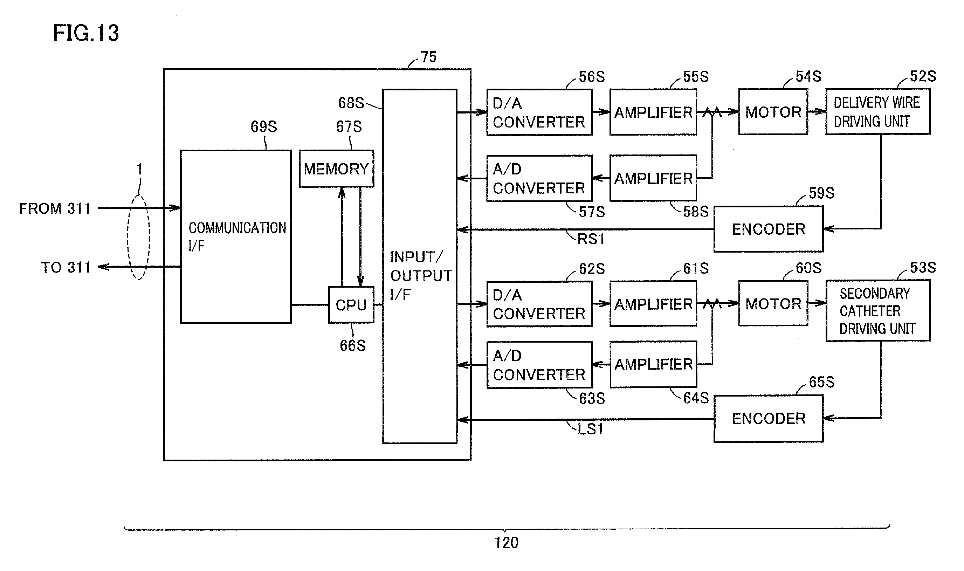

Figs. 12 and 13 represent the transfer of signals between slave 120 and master 210 as well as related portion thereto according to the present embodiment. Referring to Figs. 12 and 13, master control unit 311 of master 210 and slave control unit 75 of slave 120 are formed of microcomputers, which are connected to each other through a wire or wireless communication path 1. Communication unit 300 is provided at communication path 1, which is not illustrated.

Referring to Fig. 12, master control unit 311 includes a CPU (Central Processing Unit) 28M to control and monitor the operation of master 210 in a centralized manner, a memory 29M storing programs and data, an input/output I/F (Interface) 31M for controlling input and output with respect to other elements in master 210, a communication I/F (Interface) 30M for controlling communication with communication path 1, an external I/F (Interface) 32M for controlling input and output with an externally provided notification unit 27M, and a storage unit 33M. Storage unit 33M has a larger storage capacitance as compared to memory 29M to store image data and the like. Notification unit 27M includes a section to output sound or light, allowing light or sound to be output according to an applied signal. Input/output I/F 31M is connected with enable switch 312, as well as circuits related to each of delivery wire manipulation unit 13M and secondary catheter manipulation unit 14M.

In association with delivery wire manipulation unit 13M, a motor 15M, amplifiers (amplifier circuit) 16M and 18M, a D/A (Digital/Analog) converter 17M, and an A/D (Analog/Digital) converter 19M are connected. A digital signal output by CPU 28M via input/output I/F 31M is converted into an analog signal by D/A converter 17M, applied to amplifier 16M to be amplified, and then provided to motor 15M.

Motor 15M rotates according to the applied signal. A load is exerted to hinder the rotating operation (hereinafter, referred to as "rotation load") with respect to a rotation operation of delivery wire manipulation unit 13M.

Motor 15M is a DC motor. The current signal applied to motor 15M is detected by a current sensor or the like. The detected current signal is applied to amplifier 18M. The current signal amplified by amplifier 18M is converted into a digital signal by A/D converter 19M to be provided to CPU 28M via input/output I/F 31M.

In association with secondary catheter manipulation unit 14M, a motor 21M, amplifiers (amplify circuit) 22M and 24M, a D/A (Digital/Analog) converter 23M, and an A/D (Analog/Digital) converter 25M are connected. A digital signal output by CPU 28M via input/output I/F 31M is converted into an analog signal by D/A converter 23M, and then applied to amplifier 22M to be amplified and provided to motor 21 M.

Motor 21M rotates according to an applied signal. The aforementioned load is exerted to hinder the rotating operation with respect to a rotation operation of secondary catheter manipulation unit 14M.

Motor 21M is a DC motor. The current signal applied to motor 21M is detected by a current sensor or the like. The detected current signal is applied to amplifier 24M. Amplifier 24M amplifies the applied current signal for output to A/D converter 25M. A/D converter 25M converts the signal applied from amplifier 24M into a digital signal, which is output to CPU 28M via input/output I/F 31 M.

In the present embodiment, a rotation load is applied to both delivery wire manipulation unit 13M and secondary catheter manipulation unit 14M. However, it is not necessarily required to apply the rotation load to both manipulation units. A configuration in which the rotation load is applied to at least delivery wire manipulation unit 13M that requires more accurate manipulation may be employed.

The amount of operation on delivery wire manipulation unit 13M through manual operation of the surgeon (rotation amount (rotation angle) and rotating direction) is detected by encoder 34M. A position signal RS2 indicating the detected amount of operation is provided to CPU 28M via input/output I/F 31 M. Similarly, the amount of operation (rotation amount (rotation angle) and rotating direction) of secondary catheter manipulation unit 14M is detected by encoder 26M. A position signal LS2 indicating the detected amount of operation is provided to CPU 28M via input/output I/F 31M.

The part of slave 120 will be described with reference to Fig. 13. At slave 120, slave control unit 75 includes a CPU (Central Processing Unit) 66S for controlling and monitoring each element in slave 120 in a centralized manner, a memory 67S prestoring data and programs, a communication I/F (Interface) 69S for controlling communication with communication path 1, and an input/output I/F (Interface) 68S for controlling input and output with other elements in slave 120.

To input/output I/F 68S are connected a D/A (Digital/Analog) converter 56S, an A/D (Analog/Digital) converter 57S, amplifiers (amplify circuit) 55S and 58S, and an encoder 59S to input/output a signal to/from delivery wire driving unit 52S. Similarly, a D/A (Digital/Analog) converter 62S, an A/D (Analog/Digital) converter 63S, amplifiers (amplify circuit) 61S and 64S, and an encoder 65S are connected to input/output a signal to/from secondary catheter driving unit 53S.

A signal to drive delivery wire driving unit 52S is applied by CPU 66S to D/A converter 56S via input/output I/F 68S. D/A converter 56S converts an input signal into an analog signal, which is applied to amplifier 55S. Amplifier 55S amplifies the applied signal for output to motor 54S. Motor 54S is a DC motor, for example, to rotate according to a current signal that is the applied signal. In conjunction with this rotation, delivery wire driving unit 52S rotates.

The current signal applied to motor 54S is detected by a current sensor or the like. The detected current signal is applied to amplifier 58S. Amplifier 58S amplifies the applied current signal for output to A/D converter 57S. A/D converter 57S converts the applied current signal into a digital signal, which is applied to CPU 66S via input/output I/F 68S.

The driving amount in conjunction with motor 54S of delivery wire driving unit 52S (rotation amount (rotation angle) and rotating direction) is detected by encoder 59S. A position signal RS1 indicating the detected driving amount is applied to CPU 66S via input/output I/F 68S.

A signal to drive secondary catheter driving unit 53S is applied by CPU 66S to D/A converter 62S via input/output I/F 68S. D/A converter 62S converts an applied signal into an analog signal, which is applied to amplifier 61 S. Amplifier 61S amplifies the applied signal for output to motor 60S. Motor 60S is a DC motor, for example, rotating according to a current signal that is the applied signal. In conjunction with this rotation, secondary catheter driving unit 53S rotates.

The current signal applied to motor 60S is detected by a current sensor or the like. The detected current signal is applied to amplifier 64S. Amplifier 64S amplifies the applied current signal for output to A/D converter 63S. A/D converter 63S converts the applied signal into a digital signal, which is output to CPU 66S via input/output I/F 68S.

The driving amount in conjunction with motor 60S of secondary catheter driving unit 53S is detected by encoder 65S. A position signal LS1 indicating the detected driving amount is provided to CPU 66S via input/output I/F 68S.

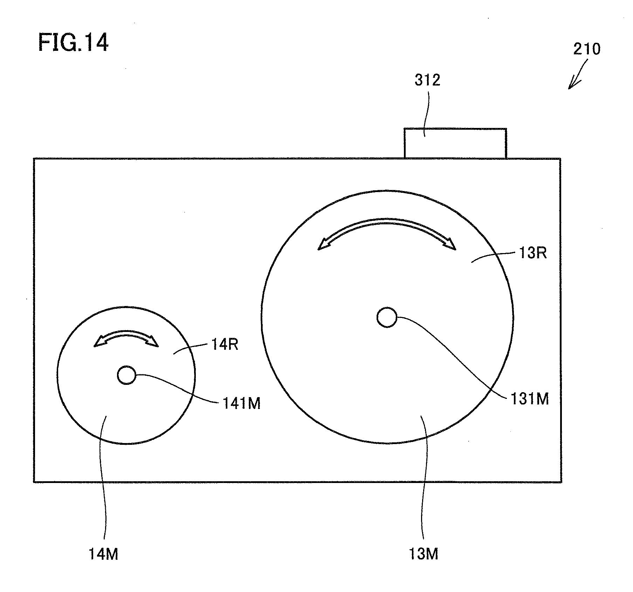

An appearance of master 210 is shown in Fig. 14. At the surface of the casing of master 210 are disposed enable switch 312, delivery wire manipulation unit 13M, and secondary catheter manipulation unit 14M. Delivery wire manipulation unit 13M and secondary catheter manipulation unit 14M are arranged in close proximity or adjacent to allow one surgeon to operate each manipulation unit with his/her left hand and right hand during manipulation. A rotator 13R of delivery wire manipulation unit 13M arranged at the surface of the casing of master 210 rotates arbitrarily in the direction of the arrow concentrically about a shaft 131 M, in conjunction with the rotating operation by the surgeon. Similarly, a rotator 14R of secondary catheter manipulation unit 14M rotates arbitrarily in the direction of the arrow concentrically about a shaft 141M, in conjunction with the rotating operation by the surgeon.

Fig. 15 schematically represents a driving mechanism of catheter 50 and delivery wire 70 on part of slave 120. Slave 120 includes an actuator 124 (refer to Fig. 11) of the roller type to move secondary catheter 372 and delivery wire 70 that are linear objects by applying an insertion force (compression force in the longitudinal axial direction acting on the linear object). The rotation control of actuator 124 is performed in conjunction with the rotary operation of the rotator of delivery wire manipulation unit 13M and secondary catheter manipulation unit 14M of master 210. A primary catheter 374 and secondary catheter 372 inserted therethrough in Fig. 15 correspond to catheter 50 in Fig. 11.

Specifically, actuator 124 connected to slave control unit 75 includes an actuator 12A for delivery wire 70 and an actuator 12B for secondary catheter 372. Actuator 12A includes a delivery wire driving unit 52S that is a roller rotating in conjunction with the rotation of motor 54S, and a roller for pressing delivery wire 70 located on the roller face of delivery wire driving unit 52S against the surface. This pressing roller rotates in conjunction with the rotation of delivery wire driving unit 52S.

Actuator 12B includes a secondary catheter driving unit 53S that is a roller rotating in conjunction with the rotation of motor 60S, and a pressing roller to press secondary catheter 372 on the roller. The pressing roller rotates in conjunction with the rotation of secondary catheter driving unit 53S.

By driving delivery wire driving unit 52S and secondary catheter driving unit 53S of actuators 12A and 12B so as to rotate in the direction of the arrow in the drawing, an insertion force is exerted on delivery wire 70 and secondary catheter 372 in conjunction with the rotation. Accordingly, delivery wire 70 and secondary catheter 372 move in a direction to be inserted into the body, or in a direction to be extracted out from the body.

Encoder 59S provided in association with delivery wire driving unit 52S and encoder 65S provided in association with secondary catheter driving unit 53S are based on a similar configuration to detect the driving amount (rotation angle and rotating direction) of delivery wire driving unit 52S and secondary catheter driving unit 53S. Here, encoder 59S will be described as a representative thereof.

Encoder 59S is, for example, a general optical rotary encoder, converting the rotation angle of the roller of delivery wire driving unit 52S using an optical sensor into a pulse train to measure the rotation angle by counting the pulse number through a counter circuit or the like, and detects the rotating direction (positive rotation/reverse rotation) based on the phase of the output waveform from the optical sensor. The encoder is not limited to the optical type, and may be a magnetic type rotary encoder.

A Y connector 371 is provided at the output stage of actuator 12A. A Y connector 373 is provided at the output stage of actuator 12B. Y connectors 371 and 373 have the function described with reference to Fig. 25. Delivery wire 70 output from actuator 12A is inserted to pass through secondary catheter 372 via Y connector 371. Secondary catheter 372 output from actuator 12B (secondary catheter 372 through which delivery wire 70 passes through) is inserted, through Y connector 373, in primary catheter 374 connected to Y connector 373. Primary catheter 374 is inserted into the body of the patient in advance. Secondary catheter 372 moving within primary catheter 374 is shifted through a vessel 840 in the body in conjunction with the rotation of secondary catheter driving unit 53S of actuator 12B, while delivery wire 70 is shifted through vessel 840 in the body in conjunction with the rotation of delivery wire driving unit 52S of actuator 12A. By this movement, the tip of delivery wire 70 arrives at a cerebral aneurysm 842 that is the target site to allow coil 53 connected at the tip of delivery wire 70 to fill cerebral aneurysm 842.

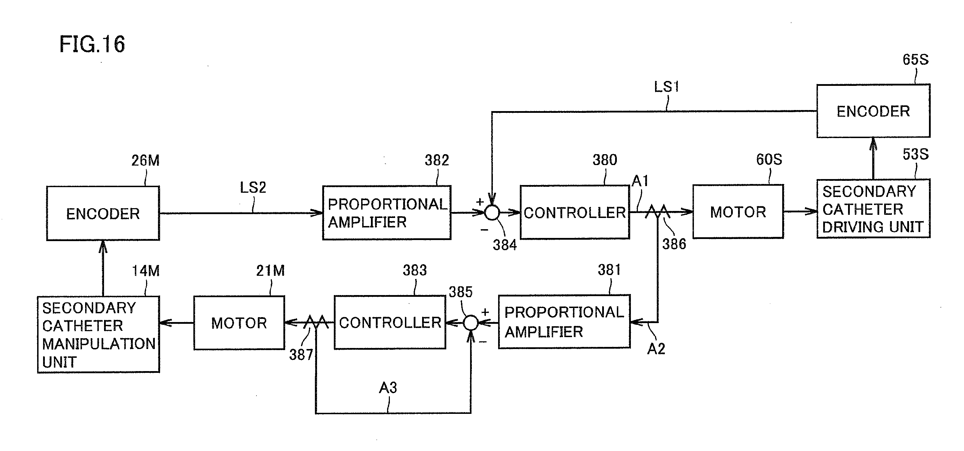

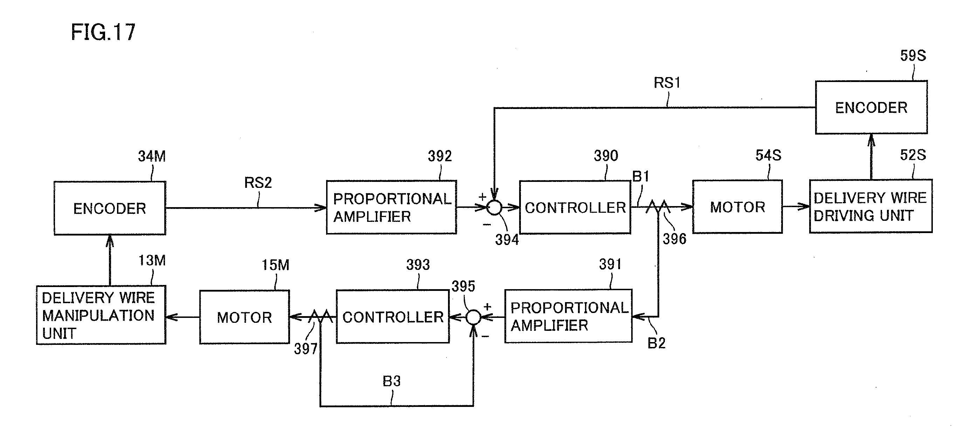

Fig. 16 schematically shows a circuit configuration to apply an insertion force to secondary catheter 372 by slave 120 (compression force in the longitudinal axial direction acting on the linear object) as the rotation load on secondary catheter manipulation unit 14M of master 210. Fig. 17 schematically represents a circuit configuration to apply the insertion force to delivery wire 70 (compression force in the longitudinal axial direction acting on the linear object) into the body by slave 120 as a rotation load on delivery wire manipulation unit 13M of master 210.

As the system for controlling the operation of secondary catheter driving unit 53S, there are provided, as shown in Fig. 16, encoders 26M and 65S, a proportional amplifier (amplify circuit) 382 amplifying an input signal according to a predetermined ratio for output, a subtracter 384 detecting a difference between applied signals for output, a controller 380, and a motor 60S for rotating secondary catheter driving unit 53S. Controller 380 corresponds to a microcomputer relevant to CPU 66S.

As the system applying rotation load to secondary catheter manipulation unit 14M, there are provided, as shown in Fig. 16, a proportional amplifier (amplify circuit) 381, a subtracter 385 detecting a difference between applied signals for output, a controller 383, and a motor 21M. Controller 383 corresponds to a microcomputer relevant to CPU 28M.

In operation, the amount of operation of secondary catheter manipulation unit 14M is detected by encoder 26M to output position signal LS2 indicating the amount of operation to proportional amplifier 382. Proportional amplifier 382 amplifies the applied position signal LS2 according to a predetermined ratio for output to subtracter 384. Subtracter 384 substracts the component of position signal LS2 applied from proportional amplifier 382 from the component of position signal LS1 applied from encoder 65S to detect the difference in component between the signals. The detected difference signal is applied to controller 380.

Controller 380 generates and outputs a drive current A1 having a current level corresponding to the difference signal between position signals LS1 and LS2 provided from subtracter 384. A table storing the data of current level corresponding to each difference signal level, for example, is prestored in memory 67S, which is referred to by CPU 66S according to the level of the difference signal. Thus, data of the corresponding current level is read out (determined).

Drive current A1 is provided to motor 60S. Motor 60S rotates in a direction and by just an amount (angle) according to drive current A1. Catheter driving unit 53S is rotated in conjunction with that rotation. Accordingly, secondary catheter 372 is applied with an insertion force according to the driving amount of secondary catheter driving unit 53S to move in the direction of insertion/extraction with respect to the body. The driving amount (rotation angle and rotating direction) of secondary catheter driving unit 53S is detected by encoder 65S to be applied to subtracter 384 as position signal LS1. Concurrently, drive current A1 is detected by current sensor 386 provided at the input stage of motor 60S. The detected drive current A1 is applied to proportional amplifier 381 as a current signal A2. Proportional amplifier 381 amplifies current signal A2 according to a predetermined ratio, and provides the amplified signal to subtracter 385. Subtracter 385 detects the difference that is the value of input current signal A3 subtracted from the current signal applied from proportional amplifier 381, and provides the detected difference signal to controller 383.

Controller 383 generates a drive signal that is a current signal of a level corresponding to the applied difference signal. For example, a table (not shown) storing data of a current level corresponding to each of the level of the difference signal is prestored, for example, in memory 29M, which is referred to by CPU 28M based on the level of the difference signal. Thus, data of the corresponding current level is read out (determined). Then, a current signal of the read out level is generated and output.

The drive signal output from controller 383 is provided to motor 21M. Motor 21 M rotates according to a rotation angle and rotating direction corresponding to the applied drive signal. Rotation load is applied to secondary catheter manipulation unit 14M in conjunction with the rotation. A current sensor 387 is provided at the input stage of motor 21M. Current sensor 387 detects the current signal applied from controller 383. The detected current signal A3 is applied to subtracter 385.

The predetermined ratio set for proportional amplifier 382 represents the ratio of the unit rotation angle of secondary catheter driving unit 53S to the rotation angle of secondary catheter manipulation unit 14M for providing the unit rotation angle.

Since secondary catheter driving unit 53S is driven such that the difference becomes zero according to the driving amount corresponding to the difference output from subtracter 384, as shown in Fig. 16, the driving amount of secondary catheter driving unit 53S linearly follows the amount of operation of secondary catheter manipulation unit 14M. Further, since motor 21M directed to applying a rotation load to secondary catheter manipulation unit 14M is driven according to the driving amount corresponding to the difference output from subtracter 385, i.e. driven such that the difference becomes zero, the rotation load applied by the rotation amount of motor 21 M linearly follows the driving amount of catheter driving unit 53S. Since the insertion force acting on secondary catheter 372 corresponds to the driving amount of catheter driving unit 53S, the surgeon can sense the level of the insertion force acting on secondary catheter 372 by perceiving the rotation load applied to secondary catheter manipulation unit 14M as operation resistance. The ratio of the insertion force acting on secondary catheter 372 to the operation resistance applied to the surgeon is determined by the predetermined ratio set at proportional amplifier 381.

Similarly, as the system for controlling the operation of delivery wire driving unit 52S, there are provided, as shown in Fig. 17, encoders 34M and 59S, a proportional amplifier (amplify circuit) 392 amplifying an input signal according to a predetermined ratio for output, a subtracter 394 detecting a difference between applied signals for output, a controller 390, and a motor 54S for rotating delivery wire driving unit 52S. Controller 390 corresponds to a microcomputer relevant to CPU 66S.

As the system for applying rotation load to delivery wire manipulation unit 13M, there are provided, as shown in Fig. 17, a proportional amplifier (amplify circuit) 391, a subtracter 395 detecting a difference between applied signals for output, a controller 393, and a motor 15M. Controller 393 corresponds to a microcomputer relevant to CPU 28M.

In operation, the amount of operation of delivery wire manipulation unit 13M is detected by encoder 34M to output a position signal RS2 indicating the amount of operation to proportional amplifier 392. Proportional amplifier 392 amplifies the applied position signal RS2 according to a predetermined ratio for output to subtracter 394. Subtracter 394 subtracts the component of amplified position signal RS2 applied from proportional amplifier 392 from the component of position signal RS1 applied from encoder 59S to detect the difference in component between the signals. The detected difference signal is applied to controller 390.

Controller 390 generates and outputs a drive current B1 having a current level corresponding to the difference indicated by the difference signal provided from subtracter 394. CPU 66S refers to a table prestored in memory 67S according to the level of the difference signal level, as set forth above, to read out (determine) data of the corresponding current level. Thus, a signal of a drive current B1 of the read out level is generated and output.

The signal of drive current B1 is provided to motor 54S. Motor 54S rotates according to a driving amount corresponding to drive current B 1. Delivery wire driving unit 52S is rotated in conjunction with that rotation. Accordingly, delivery wire 70 is applied with an insertion force according to the driving amount of drive current B1 to move in the direction of insertion/extraction with respect to the body.

The driving amount (rotation angle and rotating direction) of delivery wire driving unit 52S is detected by encoder 59S to be applied to subtracter 394 as position signal RS1. Concurrently, drive current B1 is detected by a current sensor 396 provided at the input stage of motor 54S. The detected drive current B1 is applied to proportional amplifier 391 as a current signal B2. Proportional amplifier 391 amplifies current signal B2 according to a predetermined ratio, and provides the amplified signal to subtracter 395. Subtracter 395 detects the difference that is the value of input current signal B3 subtracted from the current signal applied from proportional amplifier 391, and provides the detected difference signal to controller 393.

Controller 393 generates a drive signal corresponding to the applied difference signal. For example, CPU 28M refers to a table (not shown) prestored in memory 29M according to the level of the difference signal level, as set forth above, to read out (determine) data of the corresponding current level. Then, a current signal of the read out level is generated and output as the driving signal.

The drive signal is output to motor 15M. Motor 15M rotates according to the driving amount corresponding to the applied drive signal. A rotation load is applied to delivery wire manipulation unit 13M in conjunction with the rotation. A current sensor 397 is provided at the input stage of motor 15M. Current sensor 397 detects the current signal applied to motor 15M. The detected current signal is applied to subtracter 395 as current signal B3.

The predetermined ratio set for proportional amplifier 392 represents the ratio of the unit rotation angle of delivery wire driving unit 52S to the rotation angle of secondary catheter manipulation unit 14M for providing the unit rotation angle.

Since delivery wire driving unit 52S is driven such that the difference becomes zero according to the driving amount corresponding to the difference output from subtracter 394, as shown in Fig. 17, the driving amount of delivery wire driving unit 52S linearly follows the amount of operation of delivery wire manipulation unit 13M. Further, since motor 15M directed to applying a rotation load to delivery wire manipulation unit 13M is driven according to the driving amount corresponding to the difference output from subtracter 395, i.e. driven such that the difference becomes zero, the rotation load applied by the rotation of motor 15M linearly follows the driving amount of delivery wire driving unit 52S. Since the insertion force acting on delivery wire 70 corresponds to the driving amount of delivery wire driving unit 52S, the surgeon can sense the level of the insertion force acting on delivery wire 70 by perceiving the rotation load applied to delivery wire manipulation unit 13M as an operation resistance. The ratio of the insertion force acting on delivery wire 70 to the resistance of the operation applied to the surgeon is determined by the predetermined ratio set at proportional amplifier 391.

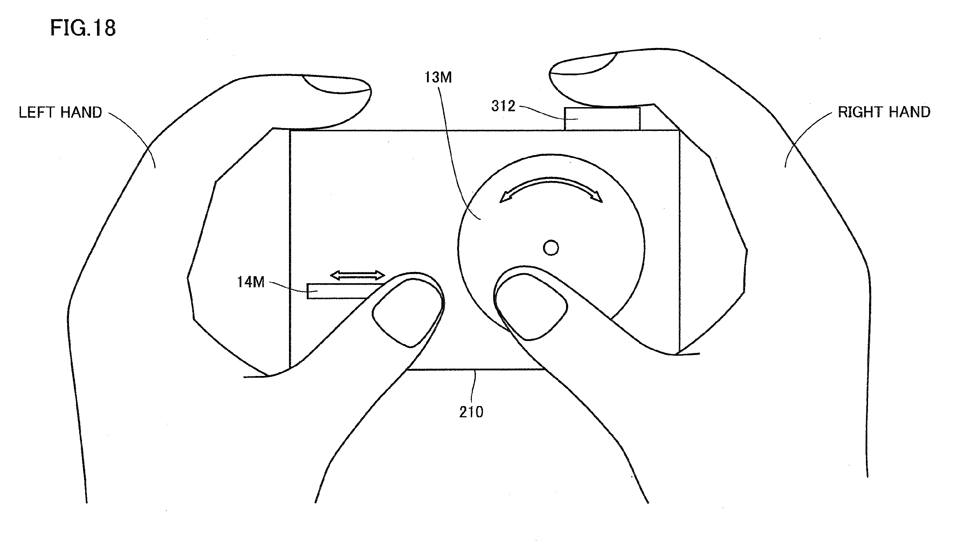

Fig. 18 shows another configuration of master 210. As the casing of master 210, the manipulation unit may be provided inclined 90° with respect to the rotation shaft. Master 210 is readily operable if it is configured to allow operation while being held by the hands, as shown in Fig. 18. Enable switch 312 is preferably configured to be depressed by one's forefinger or the like. The rotation manipulation units of delivery wire manipulation unit 13M and secondary catheter manipulation unit 14M are preferably configured to be operated with each thumb. Although the device shown in Figs. 12 and 18 includes only one enable switch 312, two enable switches may be provided alternatively, i.e. an enable switch for each of delivery wire manipulation unit 13M and secondary catheter manipulation unit 14M.

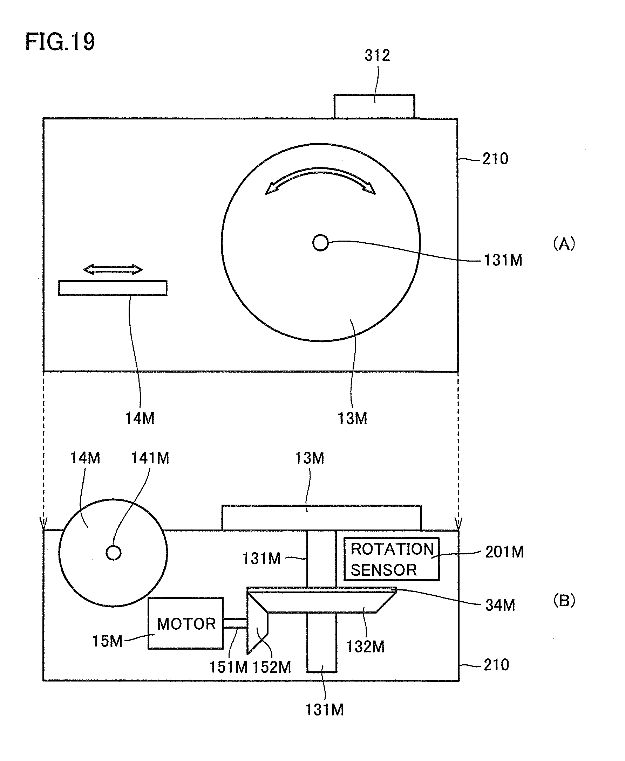

Corresponding to the configuration of the casing of master 210 shown in Fig. 19(A) which is a top view, Fig. 19 (B) shows the internal configuration of the casing. The mechanism of applying rotation load to delivery wire manipulation unit 13M will be described with reference to Fig. 19 (B). This mechanism can be applied similarly to a mechanism for applying a rotation load to secondary catheter manipulation unit 14M. The mechanism of delivery wire manipulation unit 13M will be described representative thereof hereinafter.

Referring to Fig. 19 (B), delivery wire manipulation unit 13M has rotation shaft 131M provided extending from the surface of the casing in the casing of master 210. A disk-like member 132M is attached integrally with rotation shaft 131. Rotation shaft 131 M extends so as to pass through the center of both the top plane and bottom plane of disk-like member 132M.

In operation, rotation of rotation shaft 131M in conjunction with the operation of delivery wire manipulation unit 13M causes disk-like member 132M provided integrally with rotation shaft 131 M to rotate in conformance with the amount of operation of delivery wire manipulation unit 13M. The rotating direction and rotation angle of disk-like member 132M are detected by a rotation sensor 201M provided in master 210. Rotation sensor 201M may be, for example, of the optical type. The detected result from rotation sensor 201M is applied to encoder 34M to be encoded. Accordingly, the detected result is converted (generated) into the aforementioned position signal RS2 indicative of the rotation amount and the rotating direction for output. The functions of rotation sensor 201M and encoder 34M are similar to those of the optical type rotary encoder set forth above.

In the casing of master 210 are provided a force sensing motor 15M to apply a rotation load to delivery wire manipulation unit 13M, and a disk-like member 152M. The disk-like member 152M has attached a motor shaft 151M extending from motor 15M to pass through the center of the top face and the center of the bottom face of the disk. Therefore, disk-like member 152M is rotated in a direction matching the rotating direction of motor 15M by just an angle corresponding to the rotation angle of motor 15M, via motor shaft 151M.

Disk-like member 152M is attached such that the side face of the disk (the face between the top face and bottom face of the disk) is brought into contact with the side face of disk-like member 132M. In operation, the torque to be generated at delivery wire manipulation unit 13M is adjusted by controlling the driving amount of motor 15M (the level of current supplied to motor 15M).

The direction of the velocity vector generated in the tangential direction of the disk of rotating disk-like member 132M is opposite to the direction of the velocity vector generated in the tangential direction of the disk of rotating disk-like member 152M. Accordingly, the rotation of disk-like member 152M acts to reduce the rotating speed of disk-like member 132M. As a result, a load corresponding to the driving amount of motor 15M and an insertion force of delivery wire 70 is applied to the rotary operation by the surgeon on delivery wire manipulation unit 13M.

During the operation set forth above, CPU 28M on part of master 210 detects whether an ON operation is made on enable switch 312 based on the signal input from enable switch 312 through input/output I/F 31M. The surgeon effects this ON operation by depressing enable switch 312 with his/her finger when the surgeon is to intentionally carry out manipulation control of the linear object. In the case where the surgeon is to force the linear object manipulation control to be stopped, he/she takes his/her finger off from enable switch 312 for an OFF operation. CPU 28M forces the linear object manipulation control to be stopped during detection of an ON operation with enable switch 312 being depressed by the finger. In other words, input of a signal from manipulation unit 323 is stopped. Accordingly, the operation of manipulation unit 323 is disabled. A driving signal will not be applied to delivery wire driving unit 52S and secondary catheter driving unit 53S. Any movement of delivery wire 70 and secondary catheter 372 is stopped.

When an OFF operation is made in an ON state of enable switch 312, CPU 28M responds to an external interruption signal to force the execution of a process involved in the relevant control to be stopped even during execution of linear object manipulation control since a signal of OFF operation may be applied as an external interruption signal towards CPU 28M.

Thus, when an OFF operation is made on enable switch 312 (for example, one's finger is taken off from enable switch 312), the operation of manipulation unit 323 at master 210 is disabled from a valid state. Therefore, an operation signal is not transmitted to slave 120. As a result, the operation of actuator 124 at slave 120 is stopped to immobilize the motion of delivery wire 70 and secondary catheter 372. Accordingly, any movement of delivery wire 70 and secondary catheter 372 by an unintentional operation of manipulation unit 323 can be avoided, improving the safety.

During control operation, CPU 28M may receive an X-ray image from X-ray imaging device 43 via communication unit 300 to store the received image and the data of the driving amount of actuator 124 received from slave 120 in storage unit 33M in an associated state. By recording the driving amount of actuator 124 and the X-ray image data taken during the surgical procedure in an associated state, and then reading out and reproducing the recorded contents at a later date for analysis, the procedure of the physical during a surgical process can be evaluated. Moreover, the amount of operation of manipulation unit 323 at master 210 may be recorded in an associated state.

CPU 28M provides the driving amount of actuator 124 externally through sound, light, or the like via notification unit 27M. Accordingly, the surgeon can perceive the load involved in rotation as tactile resistance, as well as through audible or visual confirmation.

Communication path 1 for master 210 and slave 120 may employ electric power, electromagnetic wave such as infrared ray, and/or a network line. For example, the surgeon can control slave 120 holding a portable (handy type) master 210 through wire communication, or through infrared ray, or have an X-ray image during the surgical procedure provided on display 40 through communication path 1, allowing a remote surgical procedure while confirming the image.

### (Second Example)

In the present example a linear object manipulation control device allowing the delivery wire to be extracted from the body with an appropriate force will be described hereinafter. In the following, the same component has the same reference character allotted. Their designation and function are also identical. Therefore, detailed description thereof will not be repeated.

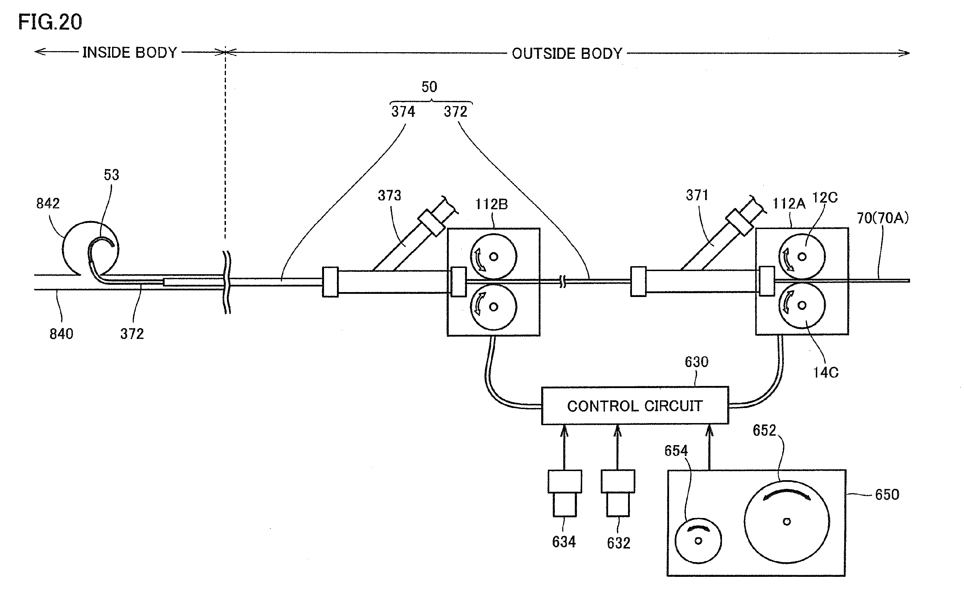

As shown in Fig. 20, a linear object extraction device in the linear object manipulation control device according to an embodiment of the present invention includes a delivery wire driving unit 112A, a secondary catheter driving unit 112B, and a control circuit 630.

By way of example, a linear object manipulation control device having an extraction function as a medical tool employed in a coil embolization treatment of cerebral aneurysm 842 formed in vessel 840 in the brain will be described in the present embodiment. The application of the extraction function is not limited to the treatment of coil embolization of cerebral aneurysm 842.

Coil 53, which is made of platinum, for coil embolization is connected at the tip of delivery wire 70. Delivery wire 70 and catheter 50 are inserted in Y connectors 371 and 373, respectively.

Catheter 50 includes a primary catheter 374 and a secondary catheter 372. Primary catheter 374 and secondary catheter 372 are hollow. Secondary catheter 372 is inserted through the hollow portion of primary catheter 374. Delivery wire 70 is inserted through the hollow portion of secondary catheter 372.

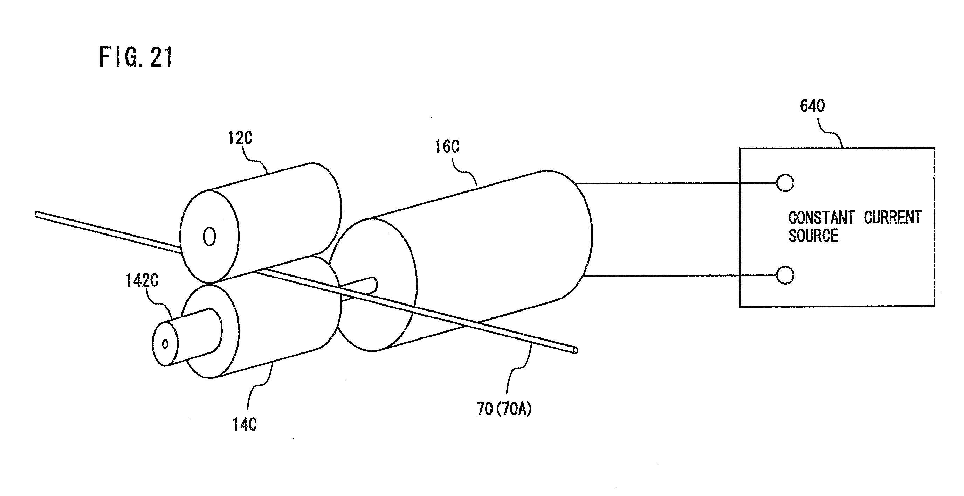

Delivery wire driving unit 112A is provided in the proximity of the entrance of Y connector 371. As shown in Fig. 21, delivery wire driving unit 112A includes a pressing roller 12C, a feeding roller 14C, and a motor 16C.

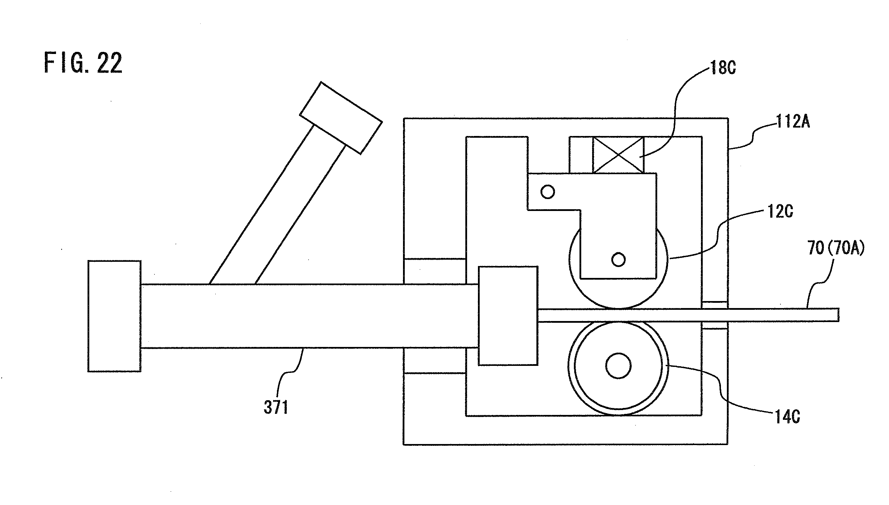

Pressing roller 12C and feeding roller 14C are provided to sandwich delivery wire 70 therebetween. As shown in Fig. 22, pressing roller 12C is supported by a resilient member 18C. By the resilience of resilient member 18C, the required force to hold delivery wire 70 between pressing roller 12C and feeding roller 14C is applied.

Returning to Fig. 21, feeding roller14C is connected to motor 16C. Feeding roller 14C is connected directly to the rotor of motor 16C without any speed reducer or the like. Motor 16C is driven by a current supplied from a constant current source 640. The driving force of motor 16C, i.e. the force of inserting or extracting platinum coil 53 (delivery wire 70), is determined according to the value of the current of motor 16C. Therefore, by determining the current value of motor 16C in the present embodiment, the force of inserting or extracting platinum coil 53 (delivery wire 70) is determined.

The number of rotations (accumulated number of rotations) and rotating speed of feeding roller 14C are detected by an encoder 142C. A signal representing the detected result is applied to control circuit 630. Control circuit 630 calculates the extracted length of delivery wire 70 by multiplying the circumference of feeding roller 14C with the number of rotations of feeding roller 14C, for example.

Secondary catheter driving unit 112B is based on a configuration similar to that of delivery wire driving unit 112A. Secondary catheter driving unit 112B is provided in the proximity of the entrance of Y connector 373.

Control circuit 630 controls delivery wire driving unit 112A and secondary catheter driving unit 112B such that delivery wire 70 and secondary catheter 372 are inserted into the body. Control circuit 630 has a master device 650 connected to control delivery wire driving unit 112A and secondary catheter driving unit 112B in order to insert delivery wire 70 and secondary catheter 372 according to an operation by the surgeon or the like.

Master device 650 includes a delivery wire manipulation unit 652 and a secondary catheter manipulation unit 654 of the dial type, for example. Control circuit 630 controls delivery wire driving unit 112A such that delivery wire 70 is inserted (advanced) and withdrawn by just a distance corresponding to the rotation amount of delivery wire manipulation unit 652. Further, control circuit 630 controls secondary catheter driving unit 112B to insert and withdrawn secondary catheter 372 by just a distance corresponding to the rotation amount of secondary catheter manipulation unit 654. Delivery wire 70 and secondary catheter 372 may be inserted manually by a surgeon or the like.

Moreover, control circuit 630 controls delivery wire driving unit 112A and secondary catheter driving unit 112B to extract delivery wire 70 and secondary catheter 372 by a predetermined force.

A delivery wire extraction button 632 and a secondary catheter extraction button 634 are connected to control circuit 630. When delivery wire extraction button 632 is operated to be turned ON, control circuit 630 controls delivery wire driving unit 112A to extract delivery wire 70 by a predetermined force. When secondary catheter extraction button 634 is operated to be turned ON, control circuit 630 controls secondary catheter driving unit 112B to extract secondary catheter 372 by a predetermined force.

Accordingly, delivery wire 70 and secondary catheter 372 can be extracted from the body safely and readily by an appropriate force. Therefore, the burden on the operator in extracting delivery wire 70 and secondary catheter 372 can be alleviated.

Control circuit 630 also controls delivery wire driving unit 112A to stop the extraction of delivery wire 70 when the extracted length of delivery wire 70 arrives at a predetermined length. Similarly, control circuit 630 controls secondary catheter driving unit 112B to stop the extraction of secondary catheter 372 when the extracted length of secondary catheter 372 arrives at a predetermined length.

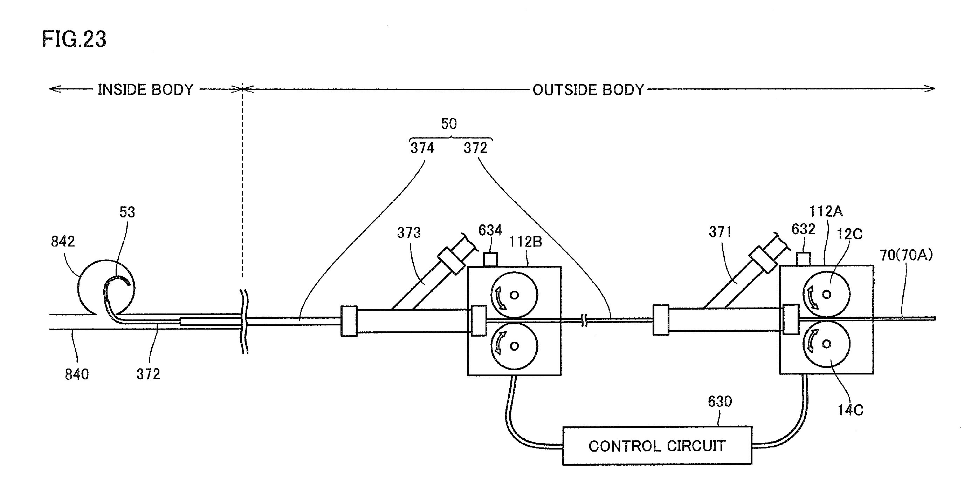

As shown in Fig. 23, delivery wire driving unit 112A and secondary catheter driving unit 112B may include delivery wire extraction button 632 and secondary catheter extraction button 634, respectively.

Moreover, the well-known guide wire 70A used in inserting catheter 50 into the body may be extracted in lieu of or in addition to delivery wire 70. Further, primary catheter 374 may be extracted in lieu of or in addition to secondary catheter 372.

Further, a plurality of extraction buttons set corresponding to different extraction lengths may be provided for one linear object. Namely, the extracting length of the linear object may be differentiated according to an operated one of the plurality of extraction buttons.

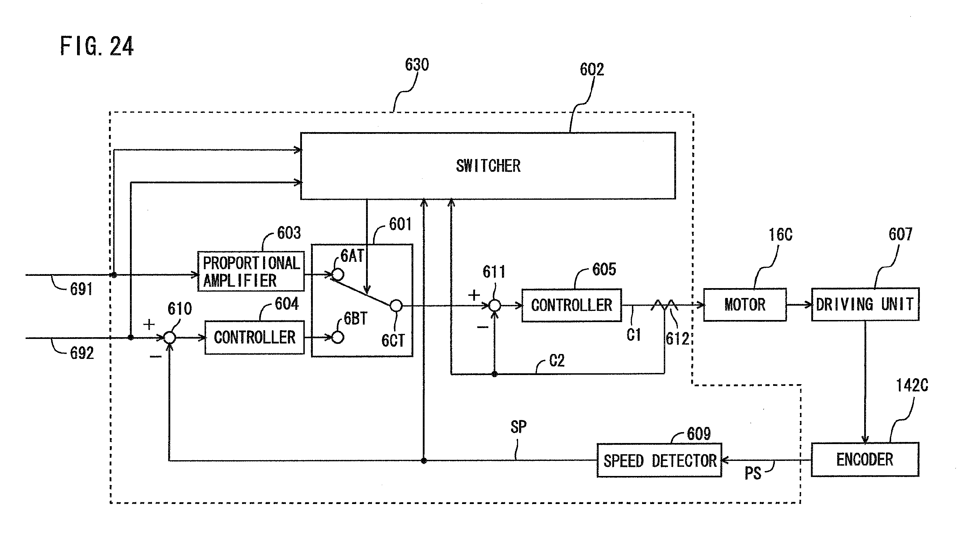

Control circuit 630 will be described further with reference to Fig. 24. Control circuit 630 includes a switch 601 and a switcher 602. Switch 601 includes input terminals 6AT and 6BT, and an output terminal 6CT. By switching the input side contact of switch 601 to either input terminal 6AT or 6BT by switcher 602, the signal output from output terminal 6CT can be switched. Accordingly, the force of extracting delivery wire 70 or catheter 372 can be modified.

Control circuit 630 further includes, for the purpose of controlling the force level and speed of extraction, a proportional amplifier 603, a subtracter 611, a current sensor 612, a controller 605, a speed detector 609, a controller 604, and a subtracter 610. Fig. 24 shows a motor 16C, a driving unit 607, and an encoder 142C as external devices that input/output a signal to/from control circuit 630. Driving unit 607 is equivalent to delivery wire driving unit 112A or secondary catheter driving unit 112B.

A instruction value signal 691 indicating the extraction force and a maximum speed signal 692 are applied to control circuit 630. A predetermined level of the force to extract the linear object, i.e. delivery wire 70 or secondary catheter 372, is designated by instruction value signal 691. The speed of extracting delivery wire 70 or secondary catheter 372 is designated by maximum speed signal 692. Instruction value signal 691 amplified by a predetermined ratio at proportional amplifier 603 is applied to input terminal 6AT of switch 601. A differential signal indicating the difference between a detection speed signal SP and maximum speed signal 692, detected by subtracter 610, is subjected to predetermined processing by controller 604, and then applied to input terminal 6BT. Detection speed signal SP indicates the moving speed of the linear object by driving unit 607. This moving speed is detected as set forth below. Encoder 142C detects the position of the linear object, and provides a position signal PS indicative of the detection result to speed detector 609. Speed detector 609 detects the moving speed based on the position indicated by position signal PS.

Referring to Fig. 24, the level of a current C1 directed to driving motor 16C output from controller 605 is detected by current sensor 612. A driving current signal C2 indicating the detected result is applied to subtracter 611. Subtracter 611 detects the difference between driving current signal C2 and the current signal output from output terminal 6CT of switch 601 to provide a signal indicating the detected difference to controller 605. Controller 605 provides current C1 of a level according to the applied difference to motor 16C. Thus, the level of the extraction force of a linear object is designated by driving current signal C2.

In the case where the contact of switch 601 is at the side of input terminal 6AT, delivery wire driving unit 112A or secondary catheter driving unit 112B is driven to extract delivery wire 70 or secondary catheter 372 by a predetermined constant force.

In the case where the contact of switch 601 is at the side of input terminal 6BT, delivery wire driving unit 112A or secondary catheter driving unit 112B is driven to extract delivery wire 70 or secondary catheter 372 at a predetermined maximum speed.

The switching of the contact of switch 601 is effected by switcher 602. For example, when the resistance against the extraction of delivery wire 70 or secondary catheter 372 is extremely low as compared with the extraction force during the extracting operation of delivery wire 70 or secondary catheter 372, delivery wire 70 or secondary catheter 372 will be extracted at high speed. If the rotating speed of the feeding roller of delivery wire driving unit 112A or secondary catheter driving unit 112B is too high, the motion of delivery wire 70 or secondary catheter 372 cannot follow the rotation, leading to the possibility of slippage between delivery wire 70 or secondary catheter 372 and the feeding roller.

In this case, the moving distance of delivery wire 70 or secondary catheter 372 based on encoder 142C that rotates integrally with the feeding roller (or motor 16C) of delivery wire driving unit 112A or secondary catheter driving unit 112B cannot be measured. Therefore, the rotation of the feeding roller must be suppressed to a level avoiding slippage between the linear object and the feeding roller.

To this end, switcher 602 switches the contact of switch 601 from input terminal 6AT to 6BT when the rotating speed of the feeding roller of delivery wire driving unit 112A or secondary catheter driving unit 112B, i.e. the speed of extracting delivery wire 70 or secondary catheter 372, is greater than a predetermined maximum speed.

Thus, the value of driving current C1 supplied to motor 16C of delivery wire driving unit 112A or secondary catheter driving unit 112B is reduced to lower the extraction force of delivery wire 70 or secondary catheter 372. The slippage between the roller feeding out the linear object and the linear object can be eliminated. As a result, calculation of the extracted length of the linear object based on the number of rotations of the feeding roller can be achieved accurately.

Further, switcher 602 switches the contact of switch 601 from terminal 6BT to terminal 6AT when the value of driving current C1 supplied to motor 16C of delivery wire driving unit 112A or secondary catheter driving unit 112B exceeds a predetermined current value, i.e. when the actual force of extracting delivery wire 70 or secondary catheter 372 exceeds a predetermined force level. Thus, the level of the extracting force of delivery wire 70 or secondary catheter 372 can be set constant.

When delivery wire 70 is present only in secondary catheter 372, the risk of damaging the body is low even if delivery wire 70 is extracted at high speed. The same applies to the case where secondary catheter 372 is located within primary catheter 374.

The function of control circuit 630 set forth above may be implemented by software or by hardware.

All the functions in the three embodiments of the linear object manipulation control device set forth above can be loaded on a single linear object control device. Not all, but one or more of the functions may be selectively loaded.

It should be understood that the embodiments disclosed herein are illustrative and non-restrictive in every respect. The scope of the present invention is defined by the terms of the claims, and is intended to include any modification within the scope of the claims.

### INDUSTRIAL APPLICABILITY

The linear object manipulation control device of the present invention can be applied to the manipulation of a medical linear object (delivery wire, catheter, guide wire, and the like).

### DESCRIPTION OF THE REFERENCE NUMBERS

1 communication path; 10, 120 slave; 11 slave control unit; 20 master; 21 master control unit; 23 delivery wire drive manipulation unit; 24 coil detachment button; 50 catheter; 51, 70 delivery wire; 53 coil; 54 connecting portion; 60 conduction switch; 62 syringe needle; 71 electrode; 80 position detection unit; 131, 373 Y connector; 210 master; 241 coil detachment start button; 242 coil detachment stop button; 300 communication unit; 312 enable switch; 372 secondary catheter; 374 primary catheter; 601 switch; 602 switcher; 630 control circuit; 632 delivery wire extraction button; 634 secondary catheter extraction button; 640 constant current source; 650 master device; 652 delivery wire manipulation unit; 654 secondary catheter manipulation unit; 701 movable electrode unit; 840 vessel; 842 cerebral aneurysm; 12C pressing roller; 14C feeding roller; 16C motor; 18C resilient member; 13M delivery wire manipulation unit; 14M secondary catheter manipulation unit; 112A delivery wire driving unit; 112B secondary catheter driving unit; 142C encoder.

## Claims

1. A linear object manipulation control device comprising:
a master (20) including an externally operable manipulation unit (23, 24), and
a slave (10) adapted to apply a force in a longitudinal axial direction to a linear object inserted in a body (41A) based on an operation through said manipulation unit to move the linear object within said body,
said master including
an operation amount detection unit (13M, 14M) adapted to detect an externally applied amount of operation of said manipulation unit, and
an operation amount transmission unit (30M) adapted to transmit the amount of operation detected by said operation amount detection unit to said slave,
said slave including
a linear object driving unit (52S, 54S, 60S, 53S) adapted to apply a force in the longitudinal axial direction to said linear object according to an applied driving signal (A1, B1),
an operation amount reception unit (69S) adapted to receive said amount of operation from said operation amount transmission unit, and
a conversion unit (56S, 62S) adapted to convert said amount of operation received from said operation amount reception unit into said driving signal for output to said linear object driving unit,
said linear object including a catheter and a wire (70) inserted in said catheter,
said manipulation unit including a catheter manipulation unit (14M) and a wire manipulation unit (13M) for operating said catheter and said wire individually,
said catheter manipulation unit and said wire manipulation unit being arranged in close proximity at a surface of a casing of said master,
**characterized in that**
said master further includes a load applying unit (15M, 21M) adapted to apply a load according to said driving signal received from said slave to said manipulation unit,
said conversion unit of said slave is adapted to convert said amount of operation received from said operation amount reception unit into said driving signal, and is adapted to output the driving signal to said linear object driving unit and said load applying unit

2. The linear object manipulation control device according to claim 1, wherein said master further includes a notification unit (27M) externally notifying said driving signal received from said slave.

3. The linear object manipulation control device according to claim 1, wherein said master is portable.

4. The linear object manipulation control device according to claim 1, wherein said master further includes an externally operated switching unit (312) at the surface of said casing for switching the operation of said manipulation unit valid or invalid.

5. The linear object manipulation control device according to claim 1, wherein
each of said catheter manipulation unit and said wire manipulation unit includes a rotator (13R, 14R) at the surface of said casing, rotationally operated arbitrarily from an external source,
said linear object driving unit includes a roller rotating according to the applied driving signal,
a force in said longitudinal axial direction is applied to said linear object in conjunction with rotation of said roller,
a level of said driving signal is proportional to a rotating amount of operation of said rotator.

6. The linear object manipulation control device according to claim 1, wherein said master and said slave communicate through wire or wirelessly.

7. The linear object manipulation control device according to claim 1, wherein said master further includes a storage unit (29M) storing said amount of operation and a level of a corresponding driving signal in association.

## Patentansprüche

1. Handhabungssteuervorrichtung für ein lineares Objekt, die Folgendes aufweist:
einen Master bzw. Geber (20), welcher eine extern bedienbare Handhabungseinheit (23, 24) aufweist, und
einen Slave bzw. Nehmer (10), welcher angepasst ist, um eine Kraft in einer longitudinalen axialen Richtung auf ein lineares Objekt auszuüben, welches in einen Körper (41A) eingeführt ist, basierend auf einer Bedienung bzw. Betätigung durch die Handhabungseinheit, um das lineare Objekt innerhalb des Körpers zu bewegen,
wobei der Master Folgendes aufweist:
eine Betätigungsbetrags-Erfassungseinheit (13M, 14M), welche angepasst ist, um einen extern ausgeübten Betätigungsbetrag der Handhabungseinheit zu erfassen, und
eine Betätigungsbetrags-Übertragungseinheit (30M), welche angepasst ist, um den Betätigungsbetrag, welcher durch die Betätigungsbetrags-Erfassungseinheit erfasst wird, zu dem Slave zu übertragen,
wobei der Slave Folgendes aufweist:
eine Antriebseinheit (52S, 54S, 60S, 53S) für ein lineares Objekt, welche angepasst ist, um eine Kraft in der longitudinalen axialen Richtung auf das lineare Objekt gemäß einem angelegten Antriebssignal (A1, B1) auszuüben,
eine Betätigungsbetrags-Empfangseinheit (69S), welche angepasst ist, um den Betätigungsbetrag von der Betätigungsbetrags-Übertragungseinheit zu empfangen, und
eine Umwandlungseinheit (56S, 62S), welche angepasst ist, um den Betätigungsbetrag, welcher von der Betätigungsbetrags-Empfangseinheit empfangen wird, in das Antriebssignal umzuwandeln zur Ausgabe zu der Antriebseinheit für das lineare Objekt,
wobei das lineare Objekt einen Katheter und einen Draht (70), welcher in den Katheter eingeführt ist, aufweist,
wobei die Handhabungseinheit eine Katheter-Handhabungseinheit (14M) und eine Draht-Handhabungseinheit (13M) zum individuellen Betätigen des Katheters und des Drahts aufweist,
wobei die Katheter-Handhabungseinheit und die Draht-Handhabungseinheit in naher Nachbarschaft an einer Oberfläche eines Gehäuses des Masters angeordnet sind,
**dadurch gekennzeichnet, dass**
der Master weiterhin eine Last-Ausübungseinheit (15M, 21M) aufweist, welche angepasst ist, um eine Last gemäß dem Antriebssignal, welches von dem Slave enthalten wird, auf die Handhabungseinheit auszuüben,
wobei die Umwandlungseinheit des Slave angepasst ist, um den Betätigungsbetrag, welcher von der Betätigungsbetrags-Empfangseinheit empfangen wird, in das Antriebssignal umzuwandeln, und angepasst ist, um das Antriebssignal zu der Antriebseinheit für das lineare Objekt und die Last-Ausübungseinheit auszugeben.

2. Handhabungssteuervorrichtung für ein lineares Objekt nach Anspruch 1, wobei der Master weiterhin eine Bekanntgabeeinheit (27M) aufweist, welche das Antriebssignal, welches von dem Slave empfangen wird, extern bekanntgibt.

3. Handhabungssteuervorrichtung für ein lineares Objekt nach Anspruch 1, wobei der Master tragbar ist.

4. Handhabungssteuervorrichtung für ein lineares Objekt nach Anspruch 1, wobei der Master weiterhin eine extern betätigte Schalteinheit (312) an der Oberfläche des Gehäuses aufweist zum zulässig- oder nicht zulässig- Schalten der Betätigung der Handhabungseinheit.

5. Handhabungssteuervorrichtung für ein lineares Objekt nach Anspruch 1, wobei
jede der Katheter-Handhabungseinheit und der Draht-Handhabungseinheit einen Rotator bzw. Dreheinrichtung (13R, 14R) an der Oberfläche des Gehäuses aufweist, welcher beliebig von einer externen Quelle drehend betrieben wird,
wobei die Antriebseinheit für das lineare Objekt eine Walze bzw. Rolle aufweist, welche sich gemäß dem angelegten Antriebssignal dreht,
wobei eine Kraft in der longitudinalen axialen Richtung auf das lineare Objekt in Verbindung mit der Drehung der Rolle ausgeübt wird,
wobei ein Pegel des Antriebssignals proportional zu einem Drehbetrag der Betätigung des Rotators ist.

6. Handhabungssteuervorrichtung für ein lineares Objekt nach Anspruch 1, wobei der Master und der Slave über einen Draht oder drahtlos kommunizieren.

7. Handhabungssteuervorrichtung für ein lineares Objekt nach Anspruch 1, wobei der Master weiterhin eine Speichereinheit (29M) aufweist, die den Betätigungsbetrag und einen Pegel eines entsprechenden Antriebssignals in Gemeinschaft speichert.

## Revendications

1. Dispositif de commande d'actionnement d'un objet linéaire comprenant :
un dispositif maître (20) incluant une unité d'actionnement opérable de façon externe (23, 24), et
un dispositif esclave (10) conçu pour appliquer une force dans une direction axiale longitudinale à un objet linéaire inséré dans un corps (41A) sur la base d'un fonctionnement par l'intermédiaire de ladite unité d'actionnement afin de déplacer l'objet linéaire à l'intérieur dudit corps,
ledit dispositif maître incluant
une unité de détection de quantité d'actionnement (13M, 14M) conçue pour détecter une quantité d'actionnement de ladite unité d'actionnement appliquée de l'extérieur, et
une unité de transmission de quantité d'actionnement (30M) conçue pour transmettre la quantité d'actionnement détectée par ladite unité de détection de quantité d'actionnement audit dispositif esclave,
ledit dispositif esclave incluant
une unité de commande d'objet linéaire (52S, 54S, 60S, 53S)) conçue pour appliquer une force dans la direction axiale longitudinale au dit objet linéaire selon un signal de commande appliqué (A1 ; B1),
une unité de réception de quantité d'actionnement (69S) conçue pour recevoir ladite quantité d'actionnement à partir de ladite unité de transmission de quantité d'actionnement, et
une unité de conversion (56S, 62S) conçue pour convertir ladite quantité d'actionnement reçue à partir de ladite unité de réception de quantité d'actionnement en ledit signal de commande à délivrer à ladite unité de commande d'objet linéaire,
ledit objet linéaire incluant un cathéter et un fil métallique (70) inséré dans ledit cathéter,
ladite unité d'actionnement incluant une unité d'actionnement de cathéter (14M) et une unité d'actionnement de fil métallique (13M) permettant d'actionner individuellement ledit cathéter et ledit fil métallique,
ladite unité d'actionnement de cathéter et ladite unité d'actionnement de fil métallique étant agencées dans une très grande proximité au niveau d'une surface d'un boîtier dudit dispositif maître,
**caractérisé en ce que**
ledit dispositif maître inclut, de plus, une unité d'application de charge (15M, 21M) conçue pour appliquer une charge ledit signal de commande reçu à partir dudit dispositif esclave à ladite unité d'actionnement,
ladite unité de conversion dudit dispositif esclave est conçue pour convertir ladite quantité d'actionnement reçue à partir de ladite unité de réception de quantité d'actionnement en ledit signal de commande, et est conçue pour délivrer le signal de commande à ladite unité de commande d'objet linéaire et à ladite unité d'application de charge.

2. Dispositif de commande d'actionnement d'un objet linéaire selon la revendication 1, dans lequel ledit dispositif maître inclut, de plus, une unité de notification (27M) notifiant de façon externe ledit signal de commande reçu à partir dudit dispositif esclave.

3. Dispositif de commande d'actionnement d'un objet linéaire selon la revendication 1, dans lequel ledit dispositif maître est portable.

4. Dispositif de commande d'actionnement d'un objet linéaire selon la revendication 1, dans lequel ledit dispositif maître inclut, de plus, une unité de commutation actionnée de l'extérieur (312) au niveau de la surface dudit boîtier en vue de commuter entre valider ou invalider le fonctionnement de ladite unité d'actionnement.

5. Dispositif de commande d'actionnement d'un objet linéaire selon la revendication 1, dans lequel
chacune de ladite unité d'actionnement de cathéter et de ladite unité d'actionnement de fil métallique incluent un système rotatif (13R, 14R) au niveau de la surface dudit boîtier, actionné en rotation de façon arbitraire à partir d'une source externe,
ladite unité de commande d'objet linéaire inclue un cylindre opérant une rotation selon le signal de commande reçu,
une force s'exerçant dans ladite direction axiale longitudinale est appliquée au dit objet linéaire en relation avec la rotation dudit cylindre,
un niveau dudit signal de commande est proportionnel à la quantité d'actionnement nécessaire pour la rotation dudit système rotatif.

6. Dispositif de commande d'actionnement d'un objet linéaire selon la revendication 1, dans lequel ledit dispositif maître et ledit dispositif esclave communiquent par fil ou sans fil.

7. Dispositif de commande d'actionnement d'un objet linéaire selon la revendication 1, dans lequel ledit dispositif maître comprend, de plus, une unité de stockage (29M) stockant, de façon associée, ladite quantité d'actionnement et le niveau d'un signal de commande correspondant.
